# EUROPEAN PATENT APPLICATION

(11) **EP 2 386 862 A1**
(43) Date of publication of application: **16.11.2011**
(21) Application number: 11176939.4
(22) Date of filing: 02.10.2003
(51) Int. Cl.: G01N 33/68, C07K 16/18, C07K 16/44, G01N 33/74, G01N 33/76

(54) **Methods and kits for diagnosing appendicitis**

(30) Priority: 09.10.2002 US 417741 P; 18.12.2002 US 434692 P; 21.04.2003 US 464471 P; 21.07.2003 US 489169 P; 18.08.2003 US 496360 P
(62) Divisional of application: 03808141.0
(71) Applicant: DMI Biosciences, Inc., Englewood, CO 80110 (US)
(72) Inventor: Bar-Or, David, Englewood, CO 80110 (US); Bar-Or, Raphael, Denver, CO 80202 (US); Winkler, James, V., Denver, CO 80237 (US); Yukl, Richard, L, Denver, CO 80210 (US)
(74) Representative: Neilson, Martin Mark

(57) **Abstract**

The invention provides an improved method and kits for diagnosing appendicitis in an animal. The method comprises determining the quantities of orthohydroxyhippuric acid and of a marker of general inflammation, such as a post-translationally modified protein, present in one or more body fluids of the animal.

## Description

### FIELD OF THE INVENTION

The invention relates to the diagnosis and/or monitoring of inflammation and the diagnosis and/or monitoring of ischemia by measuring one or more post-translationally modified proteins. This invention also relates to improved methods of diagnosing appendicitis by measuring orthohydroxyhippuric acid (OHHA) and a marker of general inflammation, including certain post-translationally modified proteins.

### BACKGROUND OF THE INVENTION

Inflammation is a cascade of events through which the body responds to a variety of injuries, infections and stresses. While the inflammatory response is critical for stress response, fending off infections and healing wounds, inflammation can also be damaging. Indeed, inflammation is an important component of the pathogenic process of many diseases and disorders. In addition, the presence of inflammation in many diseases, such as cancer, is indicative of a less favorable prognosis. Finally, in the extreme, inflammation may result in a life-threatening systemic response if not properly treated.

The current methods for the diagnosis and monitoring of inflammation are inadequate, and inflammation often remains untreated or is not treated effectively. As a consequence, considerable damage can be done to the patient or the patient's life can even be put in jeopardy. Clearly, improved methods for the diagnosis and monitoring of inflammation are needed.

Early cardiac ischemia is difficult to diagnose. Cardiac markers of cellular necrosis, such as creatine kinase isoenzyme (CK-MB), myoglobin, or troponin, are unreliable markers of transient myocardial ischemia, particularly when measured in the first 2 to 6 hours after an ischemic event. Kontos, M.C. and R.L. Jesse, Am J Cardiol, 2000. 85(5A): p. 32B-39B; Ishikawa, Y., et al., Clin Chem,1997. 43(3): p. 467-75; Brogan, G.X., Jr., et al., Acad Emerg Med, 1997. 4(1): p. 6-12; Hedges, J.R, et al., Acad Emerg Med, 1996. 3(1): p. 27-33. Patients who are examined soon after the onset of ischemic symptoms typically require prolonged observation to rule out myocardial infarction or myocardial ischemia. Gomez, M.A., et al., J Am Coll Cardiol,1996. 28(1): p.25-33; Zalenski, RJ., et al., Arch Intern Med, 1997.157(10): p. 1085-91; de Winter, R.J., et al., Ann Emerg Med, 2000. 35(2): p.113-20; Peacock, W.I., et al., Ann Emerg Med, 2000. 35(3): p. 213-20.

A novel blood assay method to measure reduced exogenous cobalt binding to human albumin in patients with myocardial ischemia has been described. Bar-Or, D., E. Lau, and J.V. Winkler, J Emerg Med, 2000.19(4): p. 311-5. The albumin-cobaltbinding (ACB) assay measures the binding capacity of exogenous cobalt to the amino terminus (N-terminus) of human albumin. Under normal conditions, transition metals, including cobalt, are tightly bound to the exposed N-terminus of albumin. Kubal, G., et al., Eur J Biochem, 1994. 220(3): p. 781-7. The ACB assay is based on observations that ischemic conditions may alter the N-terminus of albumin and rapidly reduce its binding capacity for transition metals. Berenshtein, E., et al., J Mol Cell Cardiol,1997. 29(11): p. 3025-34; Bar-Or, D., et al., Eur J Biochem, 2001. 268(1): p. 42-7. Ischemia-induced alterations to albumin would be predicted to occur minutes or hours before abnormal levels of CK-MB, myoglobin, or troponin could be detected. Despite this advantage, the ACB assay has been approved only as a test to rule out cardiac ischemia, and it would be highly desirable to have an assay that could diagnose cardiac ischemia, as well as rule it out.

Brain ischemia is currently a clinical diagnosis. Although certain biochemical markers have been described, such as Enolase, S-100 family of proteins (*e.g.*, S-100B) and others, the imaging techniques available to the clinician are more reliable and specific. A reliable and specific biochemical marker for brain ischemia would be helpful in the diagnosis and monitoring of this disease.

Low birth weight (LBW) is the leading cause of fetal and neonatal morbidity and mortality worldwide. LBW is generally accepted to indicate a weight of less than 2500 grams at delivery, and may result from a newborn being born at term but small for gestational age (SGA), being born preterm and appropriate for gestational age (AGA) or being both preterm and SGA. As such, the epidemiology of LBW is complex and multifactorial.

SGA is a statistical definition, indicating that the birth weight is less than the tenth percentile for gestational age. By definition then, 10% of newborns are SGA. In practice, some of these newborns are small and well, fulfilling their genetic growth potential, and are not at substantial risk. Other SGA newborns on the other hand are truly growth impaired, failing to meet their genetic growth potential due to a variety of factors as discussed below. These newborns are said to suffer from fetal growth restriction (FGR). In practice, some infants are presumably AGA and suffer from FGR; that is to say their weight may be at the 20^{th} percentile for gestational age, but they were genetically programmed to weigh at the 80^{th} percentile. These infants are difficult to identify in a practical sense, as there is no a priori way of knowing how much an individual "should" weigh.

FGR leads to LBW both by direct impairment of fetal growth, and often in addition by necessitating indicated preterm delivery due to compromised fetal status or associated maternal disease (*e.g.*, preeclampsia). Morbidity due to LBW and/or prematurity is varied and substantial and well documented elsewhere. Additionally, recent data have suggested that a compromised intrauterine environment can have a profound influence on health in adult life, the so-called "fetal origins of disease" or Barker hypothesis. Via these various mechanisms, the disease burden attributable to FGR is tremendous.

While the fetus/neonate is often the focus of concern in pregnancies complicated by FGR, it is important to recall that these pregnancies are also often complicated by conditions that directly threaten maternal health. Most notably, preeclampsia, whose precise pathophysiology remains obscure, has long been felt to result from placental ischemia. Preeclampsia and its complications are the leading causes of maternal mortality worldwide.

While the differential diagnosis of FGR is diverse, including chromosomal, toxic, viral and other etiologies, the majority of cases result from uteroplacental insufficiency (UPI). UPI may be associated with a variety of maternal diseases (hypertension, renal disease, systemic lupus erythematosus, antiphospholipid syndrome, thrombophilia, etc.), pregnancy complications (placental abruption, preeclampsia), or may be idiopathic. Regardless of the etiology, the presumed unifying underlying pathophysiology results from reduced placental blood flow (ischemia) in either the maternal or the fetal circulation, or both.

As a crude measure, it is known that there is a direct relationship between placental weight and fetal weight, suggesting that placental resources might control fetal growth to some extent. There are a large number of placental pathologic lesions associated with FGR. In general, these are lesions that would be expected to compromise maternal and/or fetal blood flow. The association between reduced maternal and/or fetal blood flow (ischemia) and FGR is also corroborated by alarge amount of Doppler flow data in affected pregnancies. In many cases, these abnormal Doppler flow waveforms correlate well with abnormal placental pathology.

While much is known about the pathophysiology of FGR, much remains to be understood. In the clinical setting, although various risk factors for FGR are recognized, their positive predictive values and sensitivities are limited. There can be difficulty differentiating the FGR fetus from the "SGA but well" fetus. Recognizing this difference is important to avoid unnecessary interventions on well pregnancies. Early identification of pregnancies destined to be affected by FGR might help foster appropriate follow-up. Timing of delivery is also a matter of intense interest, balancing the benefits of advancing gestation against those of continuing in an ischemic environment. Finally, on a more fundamental level, access to a clinical test to identify placental ischemia and quantify its severity might ultimately help foster appropriate treatment or even prevention.

The diagnosis of appendicitis often challenges aphysician. In particular, appendicitis is very difficult to differentiate from many other abdominal and pelvic disorders, and a fully developed picture is seldom available to the physician. Subtle and atypical signs and symptoms often lead to misdiagnosis, and diagnostic delays can lead to adverse outcomes. Jahn et al., Eur. J. Surg., 163:433-443 (1997); Andersson et al., Eur. J. Sung., 166:796-802 (2000). A recent multi-center review reported an initial misdiagnosis rate of approximately 20% for appendicitis in emergency department patients with abdominal pain and without extended observation. Graff et al., Acad. Emerg. Med., 7:1244-1255 (2000).

Previous reports suggest that an elevated leukocyte count, a nonspecific inflammatory marker, is one of the few laboratory tests consistently helpful in the initial clinical evaluation of suspected appendicitis. Berry et al., Ann. Surg., 200:567-575 (1984); Andersson et al., World J. Surg., 23:133-140 (1999). Clinicians frequently rely on a combination of the initial leukocyte count result and the clinical assessment before ordering expensive, confirmatory radiological imaging tests, such as helical or contrast computed tomography (CT). Stroman et al., Am. J. Surg., 178:485-489 (1999); Pickuth al., Eur. J. Surg., 166:315-319 (2000). Unfortunately, the initial leukocyte count has low specificity (38% to 78%) and only a moderately high sensitivity (52% to 93%) for acute appendicitis. Goodman et al., Am. Surg., 61:257-259 (1995); Bolton et al., Br. J. Surg., 62:906-908 (1975); English et al., Am. Surg., 43:399-402 (1977); Vermeulen et al., Eur. J. Surg.,161:483-486 (1995); Coleman et al., Am Surg., 64:983-985 (1998); Dueholm et al., Dis. Colon Rectum, 32:855-859 (1989); Hallan et al., Eur. J. Surg., 163:533-538 (1997). Despite its limitations, the leukocyte count continues to be the most widely used laboratory test to assist the evaluation of clinically suspected appendicitis. Andersson et al., World J. Surg., 23:133-140 (1999); Bolton et al., Br. J. Surg., 62:906-908 (1975); Vermeulen et al., Eur. J. Surg., 161:483-486 (1995); Coleman et al., Am Surg., 64:983-985 (1998); Hallan et al., Eur. J. Surg., 163:533-538 (1997).

U.S. Patent No. 5,470,750 describes a method of detecting appendicitis by determining the level of σ-hydroxyhippuric acid (orthohydroxyhippuric acid, salicyluric acid, N (2-hydroxylbenzoyl)glycine) in the urine of human patients suspected of having appendicitis. A level of σ-hydroxyhippuric acid above a threshold value indicates the probable presence of appendicitis, while a level of σ-hydroxyhippuric acid below the threshold value indicates that appendicitis is probably not present. Although the σ-hydroxyhippuric acid assay has significantly higher specificity than elevated leukocyte counts (80.6% versus 67.9%, p<0.01; see Example 9 below) for the diagnosis of appendicitis, an assay that provided even greater specificity and diagnostic accuracy would be desirable.

### SUMMARY OF THE INVENTION

The invention provides a method of diagnosing or monitoring inflammation in an animal. The method comprises determining the quantity of a post-translationally modified protein, other than phosphorylated tau, present in a body fluid of an animal. If the quantity of the post-translationally modified protein is significantly altered compared to its level in the same body fluid from normal animals, then inflammation is present. General inflammation and/or specific types of inflammation can be diagnosed or monitored.

The invention also provides methods of diagnosing or monitoring ischemia which comprise determining the quantity of a post translationally modified protein, other than phosphorylated tau, present in a body fluid from an animal. The post-translationally modified protein may be an organ-specific or tissue-specific protein (*e.g*, a heart-specific protein) for diagnosing and monitoring ischemia in an organ or tissue, a cysteinylated protein and/or a pregnancy-associated protein for diagnosing or monitoring placental ischemia, a cysteinylated protein (*e.g.*, cysteinylated albumin) for diagnosing or monitoring ischemia, and/or a phosphorylated protein constituent of a body fluid (*e.g.*, phosphorylated albumin) for diagnosing or monitoring ischemia. If the quantity of the post-translationally modified protein is significantly altered compared to its level in the same body fluid from normal animals, then ischemia is present.

The invention also provides a method of diagnosing, monitoring or predicting multiple organ failure which comprises determining the quantity of a post-translationally modified protein present in a body fluid from an animal. If the quantity of the post-translationally modified protein is significantly altered compared to its level in the same body fluid from normal animals, then multiple organ failure is present or can be expected to develop.

The invention further provides kits for quantitating post-translationally modified proteins, other than phosphorylated tau. The kits comprise one or more containers, each holding a binding partner specific for a post-translationally modified protein, and instructions describing how the binding partner(s) should be used to quantitate the post-translationally modified protein(s).

The invention also provides an improved method of diagnosing appendicitis in an animal. In this method, a first body fluid and a second body fluid are obtained from the animal. The first and second body fluids may be the same or different. It is determined (i) if the quantity of orthohydroxyhippuric acid (OHHA) present in the first body fluid of the animal is significantly elevated compared to its level in the same body fluid from normal animals and (ii) if the quantity of a marker of general inflammation present in the second body fluid of the animal is significantly altered compared to its level in the same body fluid from normal animals. Finally, the results of steps (i) and (ii) are correlated to the presence or absence of appendicitis.

The invention further provides kits for diagnosing appendicitis. In a first embodiment, the invention provides kits comprising Parts (A) and (B). Part (A) comprises at least one container holding a reagent useful for determining if the quantity of OHHA present in a body fluid of an animal is significantly elevated compared to its level in the same body fluid from normal animals. Part (B) comprises at least one container holding a reagent useful for determining if the quantity of a marker of general inflammation present in a body fluid of the animal is significantly altered compared to its level in the same body fluid from normal animals.

The invention provides another kit for diagnosing appendicitis. This kit comprises at least one container holding a reagent useful for determining if the quantity of a marker of general inflammation present in a body fluid of an animal is significantly altered compared to its level in the same body fluid from normal animals. The kit further comprises instructions directing how the reagent is to be used to diagnose appendicitis.

### BRIEF DESCRIPTION OF THE DRAWING

Figure 1: Mass spectrometer printout showing profile of albumin from a normal human.
Figures 2A-B: Mass spectrometer printouts showing a profile of albumin from a normal pregnancy (Figure 2A) and a profile of albumin from a pregnancy with placental ischemia (Figure 2B).
Figures 3A-D: Box plots showing statistical analysis comparing native albumin (Figure 3A), cysteinylated albumin (Figure 3B), glycated albumin (Figure 3C) and cysteinylated glycated albumin (Figure 3D) from normal pregnancies (1) and pregnancies with placental ischemia (2).
Figures 4A-C: Box plots showing statistical analysis of renormalized data after removal of native, glycated, cysteinylated, and cysteinylated glycated albumins. The box plots compare the renormalized data for albumin missing the C-terminal leucine (Figure 4A), albumin missing the N-terminal two amino acids (aspartic acid and alanine; Figure 4B), and albumin missing the C-terminal leucine but with homocysteine (Figure 4C) from normal pregnancies (1) and pregnancies with placental ischemia (2).
Figure 5: A diagram of the transulphuration pathway.
Figure 6: A diagram of an immunoassay for phosphorylated cardiac troponin I..
Figures 7A-7S: Bar graphs comparing the sensitivities and specificities of an assay for troponin I, an assay for phosphorylated troponin I, electrocardiogram (ECG), and various combinations thereof for diagnosing and/or ruling out acute coronary syndrome.
Figure 8: A bar graph comparing the sensitivity and specificity of an assay for phosphorylated troponin I with the ACB™ test for diagnosing and/or ruling out ischemia.
Figures 9A-9B: Mass spectrometer printouts showing profiles of albumin from a rat after bowel ischemia (Figure 9A) and from a control rat (Figure 9B).

### DETAILED DESCRIPTION OF THE PRESENTLY-PREFERRED EMBODIMENTS OF THE INVENTION

"Protein" is used herein to mean protein, polypeptide, oligopeptide, peptide and/or fragments of any of the foregoing. Also, the name of a specific protein as used herein means the protein and/or fragments of the protein. For instance, "albumin" is used here to mean the full-length protein and/or fragments of albumin, "troponin I" is used here to mean the full-length protein and/or fragments of troponin I, etc.

As used herein, "post-translational modification" means any modification of a protein that occurs after peptide bond formation. Post translational modifications include cysteinylation, phosphorylation, nitrosylation, glycosylation, acylation, isoprenylation, and removal of a limited number of amino acids. Preferred are cysteinylation and phosphorylation.

Any body fluid of an animal can be used in the methods of the invention. Suitable body fluids include a blood sample (*e.g.*, whole blood, serum or plasma), urine, saliva, cerebrospinal fluid, tears, semen, vaginal secretions, amniotic fluid and cord blood. Also, lavages, tissue homogenates and cell lysates can be utilized and, as used herein, "body fluid" includes such preparations. The body fluid may be taken from any animal. Preferably, the animal is a mammal, including dogs, cats, horses, cows and humans. Most preferably, the animal is a human.

### A. Diagnosis And Monitoring Of Inflammation And Ischemia

The invention provides methods useful for diagnosing and/or monitoring inflammation and ischemia. These methods comprise determining the quantity of one or more post-translationally modified proteins, other than phosphorylated tau, present in a body fluid of an animal.

The reason for the exclusion of phosphorylated tau is as follows. Tau is a protein which normally promotes microtubule assembly and stability in the axonal compartment of neurons. Abnormally phosphorylated tau has been found in Alzheimer's disease and in other dementias and brain disorders, and the abnormally phosphorylated tau has been found to be a marker useful in the diagnosis of these diseases. In abnormally phosphoryalted tau, there is both increasedphosphorylation (hyperphosphorylation) and phosphorylation at sites other than the normal phosphorylation sites. The reasons for the abnormal phosphorylation of tau have not been identified. See Hesse et al., Neurosci. Lett., 297(3):187-190 (2001). Tau, including all its various phosphorylated forms, has also been proposed as a marker useful in diagnosing central nervous system damage caused by, e*.g.*, ischemia. See PCT application WO 00/14546. However, it has subsequently been found that the levels of phosphorylated tau did not change in patients who had suffered ischemic strokes. See Hesse et al., Neurosci. Lett., 297(3):187-190 (2001). For the foregoing reasons, phosphorylated tau does not appear to be a marker of inflammation or ischemia, and it should not be used in the present invention to diagnose or monitor inflammation or ischemia.

### B. Measurement Of Post-Translationally Modified Proteins

Any of a wide variety of methods can be used to determine the quantities of post-translationally modified proteins present in a body fluid. Suitable techniques include mass spectrometry, specialized assays for quantitating a particular post-translational modification, binding-partner assays (*e.g.*, immunoassays), etc. Preferred are binding-partner assays.

Mass spectrometry (MS) can be used to quantitate post-translationally modified proteins. The mass of a protein will vary depending on the number and types of post-translational modifications, and the quantities of different proteins of different masses can be determined by MS. A single post-translational modification of a single protein, two or more post-translational modifications of a single protein or post-translational modifications of two or more proteins can be quantititated. Indeed, MS provides a way of identifying and quantitating many or all of the post-translationally modified proteins present in a body fluid or of many or all of the post-translational modifications of a single protein in a body fluid, and suchMS profiles will be useful for diagnosing and monitoring inflammation or ischemia.

A variety of MS analysis methods may be used in the practice of the invention, as is known in the art. For instance, a protein of interest can be isolated from a body fluid by a suitable technique, such as liquid chromatography, two-dimensional gel electrophoresis or affinity chromatography. Then, the post-translationally modified species of the protein are quantitated by any MS detection method, such as electrospray ionization MS (ESI-MS), liquid chromatographytandem MS (LC-MS), matrix-assisted laser desorption/ionization MS (MALDI-MS), MALDI time-of-flight MS (MALDI-TOF-MS), etc. See, *e.g.*, Lim et al., Analytical Biochemistry, 295: 45-56 (2001). The post-translationally modified proteins can be quantitated using standards of pure recombinant proteins, a ratio to the corresponding unmodified protein in the same body fluid, or by comparison to the same protein in the same type of body fluids from normal controls.

In addition, assays which exploit a specific type of post-translational modification can be used. For instance, for phosphorylated proteins, the phosphate groups on the proteins can be selectively labeled (by selectively blocking carboxyl groups with protecting groups in such a manner that the phosphate groups are left unblocked) with a label (*e.g.*, a radiolabel, an isotope label, a fluorescent label, a colorimetric label or an affinity label) which is then measured, all as described in U.S. Patent Application Publication No. 2002/0049307, the complete disclosure of which is incorporated herein by reference. The phosphate groups on the proteins can also be reacted with a nucleophile (*e.g.*, a thiol, amine, amide or alkanol, preferably *p*-mercaptomethylbenzoic acid or a derivative thereof) to form an adduct which is then measured (*e.g.*, immunochemically, colorimetrically or by fluorescence or chemiluminescence), all as described in PCT application WO 99/38012, the complete disclosure of which is incorporated herein by reference. Finally, the phosphate groups can be removed from a protein (*e.g.*, by hydroxide mediated β-elimination) and the protein reacted with a molecule comprising a labeled phosphate reactive group (*e.g.*, thiols, amines, amides, imides, hydroxylamines, hydrazides, hydrazines, sulfites, sulfates, sulfonamides) that selectively reacts with amino acid residues that were formerly phosphorylated, and the label subsequently measured, all as described in PCT application WO 02/066988, the complete disclosure of which is incorporated herein by reference.

Binding-partner assays are assays which employ a binding partner. A "binding partner" is any material capable of binding a post-translationally modified protein specifically. "Specifically" means that the binding partner binds a post-translationally modified protein selectively in the presence of other proteins, including other post-translationally modified proteins. For instance, the binding partner may have specificity for a portion of the post-translationally modified protein which includes the post-translational modification, for a portion of the post-translationally modified protein that does not include the post-translational modification or for a post-translationally modified amino acid residue of the post-translationally modified protein (*e.g.*, a phosphorylated serine). Binding partners include antibodies, aptamers, lectins, receptors and other proteins and molecules that can bind specifically to a post-translationally modified protein. Preferred are antibodies and aptamers and assays employing them.

Antibodies suitable for use in the invention include antisera, polyclonal antibodies, omniclonal antibodies, monoclonal antibodies, bispecific antibodies, humanized antibodies, chimeric antibodies, single-chain antibodies, Fab fragments, F(ab')₂ fragments, fragments produced by an Fab expression library, epitope-binding fragments of any of the foregoing, and complementarity determining regions (CDRs). Methods of making antibodies are well known. The antibodies can be employed in any of a wide variety of immunoassay formats. Suitable immunoassay formats include homogeneous assays, heterogeneous assays, enzyme immunoassays (*e.g.*, ELISA), competitive assays, immunometric (sandwich) assays, turbidimetric assays, nephelometric assays, etc. Any of a variety of labels and detection methods may be used. Suitable labels are known and include enzymes, radioactive labels, fluorescent labels, chemiluminescent labels, bioluminescent labels, colorimetric labels, affinity labels, metal colloid labels, latex and silica particles with dyes incorporated into them and dye particles. The antibodies can be labeled to quantitate the post-translationally modified proteins or a labeled secondary or tertiary antibody or other antibody-binding compound (*e.g.*, protein A or protein G) can be used to quantitate the post-translationally modified proteins. The immunoassays may be performed manually or with an automated analyzer.

Preferred is an enzyme immunoassay. For instance, an enzyme immunoassay may be performed as follows. An antibody specific for an unmodified epitope of a post-translationally modified protein in a body fluid is immobilized on a solid surface. Suitable solid surfaces are well known and include glass, glass filters, polystyrene, polypropylene, polyethylene, nylon, polyacrylamide, nitrocellulose, agarose, and Hydrogel. The body fluid is contacted with the immobilized antibody. After washing, the post-translationally modified protein bound to the solid surface by the antibody is reacted with a second antibody or mixture of antibodies specific for an epitope containing the post-translationally modified amino acid residue(s). The second antibody(ies) can be labeled to quantitate the post-translationally modified protein or a labeled third antibody or other compound which can bind to the second antibody(ies) (*e.g.*, protein A or streptavidin) can be used to quantitate the post-translationally modified protein.

Yet another version of an enzyme immunoassay can be performed in which an antibody specific for an epitope containing post-translationally modified amino acid(s) is immobilized on the solid surface. After washing, the post-translationally modified protein bound to the solid surface is reacted with an enzyme-labeled antibody specific for an unmodified epitope on the post-translationally modified protein to quantitate it.

As another alternative, the post-translationally modified proteins could first be separated from the other constituents of the body fluid by, *e.g.*, affinity chromatography. For affinity chromatograpy, antibodies directed to an epitope containing the post-translationally modified amino acid residue(s) are attached to a solid surface (*e.g.*, beads in a column) and used to bind the post-translationally modified protein in the fluid. After washing the solid surface, the post-translationally modified proteins are eluted and measured (*e.g.*, by one of the methods described above, bymeasuringthe absorbance at 280 nm or by another method).

Aptamers can be used in place of, or in combination with, the antibodies in any of the above described assays or in other assays that employ antibodies. Aptamers are oligonucleotides that are specific for proteins, peptides, derivatives ofproteins and peptides, inorganic molecules and other non-nucleotide molecules. See, *e.g.*, PCT applications WO 00/70329, WO 01/79562 and WO 99/54506 and U.S. Patent No. 5,756,291, the complete disclosures of which are incorporated herein by reference. Aptamers suitable for use in the present invention can be prepared using the methods described in these references. Briefly, a heterogenous population of oligonucleotides of random sequences is synthesized, and a post-translationally modified protein is mixed with the heterogenous population of oligonucleotides. Complexes are formed with some, but not all, sequences present in the oligonucleotide population. The complexes are isolated, and the oligonucleotides recovered and amplified (*e.g.*, by PCR). The resulting mixture of oligonucleotides can be used as the starting material for another round of complexation, isolation and amplification, and the process will typically be repeated several times until an aptamer of satisfactory specificity is obtained and/or until a consensus aptamer sequence is identified. Suitable labels for aptamers include dyes, enzymes, radioactive labels, etc.

In assays for quantitating phosphorylatedproteins, a phosphatase inhibitor or cocktail of phosphatase inhibitors is preferably used to prevent dephosphorylation ofphosphorylated proteins. Suitable phosphatase inhibitors are known and are available commercially. For instance, cocktails of phosphatase inhibitors are available from Sigma, St. Louis, MO. The use of phosphatase inhibitors is particularly important if the body fluid must be stored for a time before the assay is performed.

In the case of the direct measurement of cysteinylated proteins, the use of reducing agents or reducing conditions should be avoided. However, it is also possible to quantitate the cysteinylated proteins by liberating the cysteine from the protein using a reducing agent or reducing conditions and then quantitating the liberated cysteine by any of the methods described above.

In the assays just described, the quantity of a post-translationally modified protein in a body fluid is determined. Any method of quantifying the post-translationally modified protein may be used. For instance, the quantity may be an amount (*e.g.*, µg) or a concentration (*e.g.*, µM), either of which is typically determined by reference to one or more standards (*e.g.*, a purified recombinant protein that has been post-translationally modified by the same post translational modification(s) as the protein being assayed). The quantity may also be a ratio or percentage compared to another compound, such as the corresponding unmodified protein in the same body fluid or the same post-translationally modified protein in the same type of body fluid from normal controls. Once the quantity of a post-translationally modified protein in a body fluid is determined, then it is determined if the quantity is significantly altered compared to its level in the same body fluid from normal animals. "Significantly" means statistically significant, and suitable methods of statistical analysis are well known in the art.

### C. Diagnosis And Monitoring Of Inflammation

Inflammation is a cascade of events through which the body responds to a variety of injuries, infections and stresses. The inflammatory response differs depending on the type, scale and location of the insult. In most cases, inflammation is marked by the recruitment of inflammatory cells, such as macrophages and neutrophils. These cells are involved in the release of inflammatory cytokines. These and other secreted factors lead to the further accumulation of inflammatory cells. The effects of this inflammatory cascade may be highly localized or, in the extreme, may result in a life-threatening systemic response. Whether local or widespread, inflammation fundamentally changes the biochemical pathways of affected areas.

In particular, it has been found that the post-translational modifications of proteins which are present in areas of inflammation, as eitherparticipants in the inflammatoryprocess or as innocent bystanders, are altered, and the proteins whose post-translational modifications are altered represent useful biomarkers of inflammation. By "altered" is meant any change or combination of changes in the level of one or more post-translational modifications and/or in the type(s) of post-translational modification(s). For instance, a protein may be post-translationally modified for the first time, may have its level of a particular post-translational modification increased, decreased or eliminated, may be modified by additional or different types of post translational modifications, etc.

### 1. Diagnosis And Monitoring Of General Inflammation

In one embodiment ofthe invention, the presence, absence or degree of inflammation in an animal is determined. This embodiment is referred to herein as the general inflammation embodiment to distinguish it from the embodiment of the invention which measures specific types of inflammation. By "specific types of inflammation" is meant inflammation of a particular tissue or organ (*e.g.,* cardiac inflammation) or inflammation present in a specific disease, condition or disorder (*e.g,* inflammation accompanying myocardial infarction, adult respiratory distress syndrome or an autoimmune disease). The general inflammation embodiment of the invention does not identify a specific location or cause of the inflammation and does not provide a diagnosis of any specific disease. However, the presence, absence or degree of inflammation may be important in making, confirming or ruling out a diagnosis of a disease and/or in determining a course of treatment for the disease. For instance, the presence, absence or degree of inflammation as determined by the general inflammation embodiment of the invention, in combination with other diagnostic parameters may be very helpful in diagnosing many diseases. As used herein, "diagnostic parameters" means laboratory findings and clinical findings (including measurements, observations and/or symptoms).

The general inflammation embodiment of the invention can also be used to monitor the course of inflammation in an animal, including determining the effectiveness of treatments of the inflammation. This should be extremely helpful in numerous diseases, especially diseases involving widespread inflammation, such as sepsis. If a particular treatment is not effective in reducing the inflammation, the treatment can be modified. For instance, the dosage of a drug can be increased or another treatment can be substituted for the ineffective treatment. The method of the invention can also be used to choose the lowest effective dose of a drug and the shortest time during which the drug needs to be administered, which is important since many anti-inflammatory drugs have serious adverse side effects when used in high doses and/or for prolonged periods of time. Finally, this embodiment of the method of the invention can be used to monitor the effectiveness of new potential treatments of inflammation, as well as known treatments.

To measure general inflammation, all of the post-translationally modified proteins, a subset ofthe post-translationally-modified proteins or a single post-translationally modified protein present in a body fluid are measured. Each post-translationally modified protein which is employed in the measurement of general inflammation is referred to herein as a "protein marker of general inflammation." Preferably, general inflammation is measured by measuring one or more post-translationally modified proteins which are constituents of a body fluid, and these proteins are the preferred protein markers of general inflammation. Preferably, the body fluid is blood since blood comes into contact, or communicates, with essentially all areas of the body. The protein constituents of blood include albumin and immunoglobulins. The blood sample is preferably serum or plasma. The most preferred protein marker of general inflammation is post-translationally modified albumin, and the preferred post-translational modifications are phosphorylation and cysteinylation.

### 2. Diagnosis And Monitoring Of Specific Types Of Inflammation

The present invention can also be used to diagnose and monitor specific types of inflammation. In particular, inflammation of a particular organ or tissue can be diagnosed and/or monitored, and inflammation associated with (*i*.*e*., present in, involved in, causing or exacerbating) a specific disease, condition or disorder can be diagnosed and/or monitored. This is accomplished by measuring the amount(s) of one or more post-translationally modified indicator protein(s) present in a body fluid. As used herein, "indicator" proteins are proteins from a particular tissue or organ ("tissue-specific" or "organ-specific" proteins) or proteins associated with a specific disease, condition or disorder ("disease-specific" proteins).

Inflammation can afflict essentially any organ or tissue in the body. Also, it is present in, is involved in, causes or exacerbates numerous diseases, disorders and conditions, including adult respiratory distress syndrome (ARDS), allergies, arthritis, asthma, autoimmune diseases (*e.g.*, multiple sclerosis), bronchitis, cancer, cardiovascular disease, chronic obstructive pulmonary disease, Crohn's disease, cystic fibrosis, emphysema, endocarditis, gastritis, graft-versus-host disease, infections (*e.g.*, bacterial, viral and parasitic), inflammatory bowel disease, injuries, ischemia (heart, brain, placental, etc.), multiple organ dysfunction syndrome (multiple organ failure), nephritis, neurodegenerative diseases (*e.g*., Alzheimer's disease and Parkinson's disease), ophthalmic inflammation, pain, pancreatitis, psoriasis, sepsis, shock, transplant rejections, trauma, ulcers (*e.g.*, gastrointestinal ulcers and ulcerative colitis), and many others. Many suitable indicator proteins are known. The following are some examples of indicator proteins:
1. For myocardial infarction - troponin I, troponin T, creatinine phosphokinase (CPK), its MB isoenzyme (CPKMB), and myoglobin.
2. For cerebral ischemia - an S100 protein, such as S100B, and enolase
3. For Alzheimer's disease - β-amyloid.
4. For Parkinson's disease - α-synuclein.
5. For multiple sclerosis - β-amyloid and myelin basic protein.
6. For hepatitis C - albumin and liver enzymes.
7. For cancer - brca1, cea, psa, etc.
8. For chronic obstructive pulmonary disease - α1-antitrypsin and surfactant proteins.
9. For asthma - α1-antitypsin and surfactant proteins.
10. For adult respiratory distress syndrome - surfactant proteins and elastase.
11. For autoimmune diseases - Rheumatoid factor, collagen, and elastase.
12. For multiple organ failure - albumin and elastase.
13. For sepsis - lipopolysaccharide binding proteins.
14. For eclampsia and preeclampsia - angiotensin and erythropoietin.

The presence, absence or degree of a specific type of inflammation provides valuable information for making, confirming or ruling out a diagnosis of a disease and/or in determining a course of treatment. It may be possible to make a diagnosis of a specific disease using only the results of the determination of the presence, absence or degree of a specific type of inflammation. However, it may be necessary or desirable to also consider other diagnostic parameters to make the diagnosis. For instance, the use of additional diagnostic parameters may improve the sensitivity and/or specificity of, and/or improve the confidence in, the diagnosis.

This embodiment of the invention can also be used to monitor the course of a specific type of inflammation, including determining the effectiveness of treatments of the inflammation. If a particular treatment is not effective in reducing the inflammation, the treatment can be modified. For instance, the dosage of a drug can be increased or another treatment can be substituted for the ineffective treatment. The method of the invention can also be used to choose the lowest effective dose of a drug and the shortest time during which the drug needs to be administered, which is important since many anti-inflammatory drugs have serious adverse side effects when used in high doses and/or for prolonged periods of time. Finally, this embodiment of the method of the invention can be used to monitor the effectiveness ofnewpotential treatments of specific types of inflammation, as well as known treatments.

### 3. Diagnosis And Monitoring Of Inflammation By Measuring Phosphorylated Proteins

A preferred post-translational modification for diagnosing and/or monitoring both general inflammation and specific types of inflammation is phosphorylation. Many of the phenomena characteristic of inflammation are associated with intensified signal transduction at the cellular and molecular levels. One important characteristic feature of cell signaling is phosphorylation. Enzymes, proteins, peptides and other molecules are activated by phosphorylation or dephosphorylation pathways. In many instances, when an activation event involves phosphorylation, it is followed by a counter-dephosphorylation step so that a pulse signal is transmitted as opposed to a continuous stimulus. A balance exists between the phosphorylases/kinases and the phosphatases. On a molecular level, inflammation can be viewed as an imbalance in favor of the kinases. Inflammation involves expression of kinases intracellularly and externalization or activation of kinases on the outer membranes of cells and in some cases, inhibition of phosphatases, resulting in increased kinase activity. Proteins which are present in the area of inflammation, as either participants in the inflammatory process or innocent bystanders, have the potential of becoming substrates for phosphorylation by the increased kinase activity (whether membrane-bound or circulating/soluble), and the phosphorylated proteins represent useful biomarkers of inflammation.

Thus, in a preferred embodiment of the present invention, inflammation is diagnosed and/ormonitoredby determining the quantity of one or more phosphorylated proteins present in a body fluid. If the quantity of the phosphorylated protein(s) is significantly increased as compared to the level in the same fluid from normal controls, then the patient is suffering from inflammation. The protein(s) can be one(s) which has(have) been phosphorylated initially at one or more sites or one(s) whose amount of phosphorylation has been increased as a result of the increased kinase activity caused by the inflammation. All of the phosphorylated proteins present in the body fluid, a single phosphorylated protein present in the body fluid, or some subset of phosphorylated proteins present in the body fluid can be measured to diagnose and/or monitor general inflammation. One or more phosphorylated indicator proteins present in one or more body fluids can be measured to diagnose and/or monitor specific types of inflammation.

Many methods can be used to quantitate the phosphorylated proteins (see above). Preferably, the phosphorylated proteins present in the body fluid are quantitated by a binding-partner assay, most preferably by an immunoassay. Many suitable immunoassay formats are known in the art. Preferably, antibodies directed to one or more epitopes comprising a phosphorylated serine residue, a phosphorylated threonine residue, or a phosphorylated tyrosine residue of the proteins are employed. Preferably, antibodies directed to the phosphorylated serine residues, phosphorylated threonine residues, and/or phosphorylated tyrosine residues of the proteins that react with any protein comprising such phosphorylated amino acids are employed. More preferably, antibodies directed to all three types of phosphorylated amino acid residues are employed. The antibodies can be labeled to quantitate the phosphorylated proteins or a labeled secondary antibody or other antibody binding compound (*e.g.*, protein A or protein G) can be used to quantitate the phosphorylated proteins. Many suitable labels are known and include enzymes, radio labels, isotopic labels, fluorescent labels, chemiluminescent labels, colorimetric labels and affinity labels. For instance, the antibodies directed to the phosphorylated amino acid residue(s) can be labeled with labels that are not detectable unless the antibody binds to the phosphorylated proteins (*i.e.,* a homogeneous immunoassay).

As noted above, all of the phosphorylated proteins present in the body fluid, a single phosphorylated protein present in the body fluid, or some subset of phosphorylated proteins present in the body fluid can be measured to diagnose and/or monitor general inflammation. In a first embodiment of the method of the invention for measuring general inflammation, all of the phosphorylated proteins present in a body fluid are measured. Preferably, the body fluid is serum or plasma. The phosphorylated proteins may be measured without separation from the other constituents of the body fluid or the phosphorylated proteins may be separated from the other constituents of the body fluid by, *e.g.*, affinity chromatography. For instance, affinity chromatograpy can be performed using antibodies directed to the phosphorylated amino acid residue(s) attached to a solid surface (*e.g.*, beads in a column) which is used to bind the phosphorylated proteins in the fluid. After washing the solid surface, the phosphorylated proteins are eluted and measured (*e.g.*, by one of the methods described above, by measuring the absorbance at 280 nm or by another method).

In a second and preferred embodiment of the method of the invention for measuring general inflammation, a single phoshorylated protein, preferably phosphorylated albumin, present in a body fluid, preferably serum or plasma, is measured. Prior to the present invention, albumin was not known to be phosphorylated *in vivo.* It is quite surprising, therefore, that the amount of phosphorylated albumin can be used to diagnose and/or monitor inflammation.

Phosphorylated albumin can be measured in a number of ways, but it is preferably measured by a binding-partner assay, most preferably an immunoassay. Many suitable immunoassay formats are known. Preferredis an enzyme immunoassay in which an antibody specific for the albumin in the body fluid (*i.e.*, an anti-human albumin antibody when the body fluid is a human body fluid, an anti-dog albumin antibody when the body fluid is a dog body fluid, etc.) is immobilized on a solid surface. The body fluid is contacted with the immobilized antibody. After washing, the albumin bound to the solid surface by the antialbumin antibody is reacted with a second antibody or mixture of antibodies specific for phosphorylated serine residues, phosphorylated threonine residues, and/or phosphorylated tyrosine residues. More preferably, antibodies directed to all three types of phosphorylated amino acid residues are employed. The second antibody(ies) canbe labeled to quantitate the phosphorylated albumin or a labeled third antibody or other compound which can bind to the second antibody(ies) *(*e*.g.*, protein A or streptavidin) can be used to quantitate the phosphorylated albumin.

Another version of this enzyme immunoassay can be performed in which the antibody or mixture of antibodies specific for phosphorylated serine residues, phosphorylated threonine residues, and/or phosphorylated tyrosine residues is immobilized on the solid surface and, after washing, the phosphorylated proteins bound to the solid surface are reacted with an antibody specific for albumin to quantitate the phosphorylated albumin.

Yet another version of this enzyme immunoassay can be performed in which an antibody specific for the phosphorylated albumin (*i.e.*, an antibody specific for a epitope of albumin comprising a phosphorylated amino acid residue) is immobilized on the solid surface. After washing, the phosphorylated albumin bound to the solid surface is reacted with an enzyme-labeled antibody specific for albumin to quantitate the phosphorylated albumin. Of course, the two antibodies could be reversed, with the antibody specific for albumin being immobilized on the solid surface and the antibody specific for the phosphorylated albumin being used as the detecting antibody.

As another alternative, albumin can first be separated from the other constituents of the body fluid by, *e.g.*, affinity chromatography. For the affinity chromatography, an antibody specific for the albumin (*e.g.*, antibodies specific for epitopes which are phosphorylated and/or antibodies specific for epitopes which are not phosphorylated) present in the body fluid is attached to a solid surface and used to bind the albumin. After washing the solid surface, the albumin is eluted and measured (*e.g.*, by one of the methods described above for albumin or by another method).

In a third embodiment of the method of the invention for measuring general inflammation, a subset of the phosphorylated proteins present in a body fluid is measured. The measurement of the phosphorylated proteins can be accomplished in a number of ways. For instance, affinity chromatography could be performed to separate all of the phosphorylated proteins from the other constituents present in the body fluid. Alternatively, an enzyme immunoassay could be performed by immobilizing antibodies specific for phosphorylated serine residues, phosphorylated threonine residues, and/or phosphorylated tyrosine residues on a solid surface to capture all of the phosphorylated proteins in the body fluid. In either case, a subset of proteins could be measured using a cocktail of antibodies specific for the chosen proteins.

To measure specific types of inflammation, one or more specific phosphorylated indicator protein(s) present in a body fluid is(are) quantitated. The one or more phosphorylated indicator proteins can be measured by one or more of the methods described above. Preferably, the phosphorylated indicator proteins are measured by a binding-partner assay, more preferably an immunoassay, most preferably an enzyme immunoassay, using appropriate antibodies (*e.g,* antibodies specific for the indicator protein(s) (epitopes which are phosporylated and/or epitopes which are not phosphorylated) and/or antibodies specific for phosphorylated serine, threonine and/or tyrosine residues).

### 4. Diagnosis And Monitoring Of Inflammation By Measuring Cysteinylated Proteins

Another preferred post-translational modification for diagnosing and/or monitoring inflammation is cysteinylation. The levels of cysteinylated proteins are thought to be increased in inflammation for at least the following reasons.

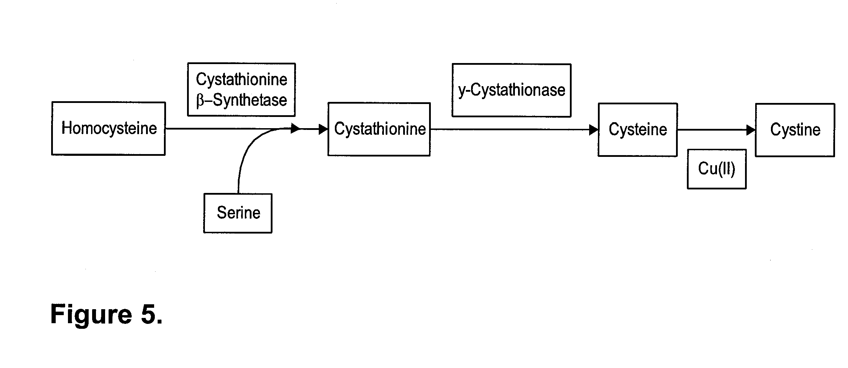

First, cystathionine β-synthetase (CBS) is present in many organs and tissues, including brain, liver, pancreas, kidney and placenta. It is also present in fetal tissues. Inflammation in these organs and tissues, or portions thereof, or in fetal tissues results in the inhibition of CBS, leading to the accumulation of homocysteine, which then enters the bloodstream and areas that are not inflammed. This homocysteine is processed by uninhibited CBS in uninflammed areas (*e.g.*, in the blood and uninflammed areas of inflammed organs and tissues) into cystathionine and cysteine through the transulphuration pathway (see Figure 5) which cysteinylates proteins.

In addition, the pH and oxygen level are decreased in inflammation, resulting in a release of Cu(II) from proteins to which it is normally bound. The Cu(II) levels are increased in both the bloodstream and in organs and tissues, so cysteinylation of proteins should be further increased during inflammation due to the increased Cu(II) levels (see Figure 5).

Thus, the cysteinylated proteins can include circulating proteins, such as albumin, which are protein markers of general inflammation. The cysteinylated proteins can also include proteins produced in uninflammed areas of an inflammed organ or tissue or of a fetus which are, therefore, useful for the diagnosis and monitoring of specific types of inflammation. The latter type of cysteinylated proteins can include cysteinylated brain proteins (such as S100 proteins) and cysteinylated placental and fetal proteins (such as β-human chorionic gonadotropin, α-fetoprotein, and pregnancy-associated protein 1A).

Cysteinylated proteins can be measured by one or more of the methods described above. Preferably, the cysteinylated proteins are measured by a binding-partner assay, more preferably an immunoassay, most preferably an enzyme immunoassay, using appropriate antibodies (*e.g,* antibodies specific for the cysteinylated protein(s) (epitopes which are cysteinylated and/or epitopes which are not cysteinylated)).

### 5. Inflammation Panels

In another embodiment of the invention, two or more post-translationally modified proteins are quantitated to improve the diagnosis and/or monitoring of inflammation and/or to provide a more complete profile of the inflammation. For instance, two or more protein markers of general inflammation in one or more body fluids could be measured to improve the diagnosis and/or monitoring of general inflammation. Similarly, the quantities of two or more indicator proteins in one or more body fluids could be determined to improve the diagnosis and/or monitoring of a specific type of inflammation and/or to determine if there is more than one specific type of inflammation present in the animal. Finally, one or more protein markers of general inflammation in one or more body fluids and one or more indicator proteins in one or more body fluids could be measured to determine the presence, absence or degree of inflammation in the animal (general inflammation determination) and, if inflammation is present, the location(s) of the inflammation and/or the disease(s) with which the inflammation is associated.

The quantitation of the post-translationally modified proteins can be accomplished by any of the methods described above. For instance, mass spectrometry could provide a profile of the inflammation by identifying and quantitating the post translationally modified proteins in one or more body fluids. Preferably, however, a collection of binding partners specific for the post-translationally modified proteins of interest will be provided. Such a collection may be a collection of test tubes, each holding a binding partner, or a microtiter plate, each well of which contains a binding partner, or an array of binding partners on a substrate. The collection of binding partners is then employed to prepare a profile of the inflammation.

### 6. Diagnosis And Monitoring Of Disease By Use Of a Combination Of A Level Of A Post Translationally Modified Protein And Other Diagnostic Parameters

As noted above, the general inflammation embodiment of the invention does not identify a specific location or cause of inflammation and does not provide a diagnosis of any specific disease. Even the determination that there is one or more specific types of inflammation often does not result in a definitive diagnosis of a disease. To diagnose a disease, it is generally necessary to use the presence, absence or degree of general and/or specific types of inflammation in combination with other diagnostic parameters. For instance, a determination that the lungs of an animal are inflammed may not be sufficient to diagnose one of several possible diseases that may be responsible for the lung inflammation. A medical history and other laboratory and clinical findings may be needed to distinguish, *e.g.*, asthma from a respiratory infection.

### D. Diagnosis And Monitoring Of Ischemia

The invention also provides methods of diagnosing and monitoring ischemia. Ischemia is defined herein to mean reduced oxygen levels in one or more tissues or organs. Ischemia may be caused by reduced blood flow or by the reduced oxygen carrying capacity of the blood. For instance, ischemia may be caused by anemia (reduced red blood cells or hemoglobin in the tissue or organ which, in turn, may be caused by reduced or blocked arterial blood flow to the tissue or organ, or by hemorrhage), hypoxia (lower than normal levels of oxygen in inspired gases, blood or tissues) or anoxia (absence or almost complete absence of oxygen in inspired gases, blood or tissues).

Ischemia may afflict a single tissue or organ or may affect a plurality oftissues and/or organs. Specific types of ischemia include cardiac ischemia, cerebrovascular ischemia, placental ischemia, bowel ischemia, etc.

As noted above, inflammation is present in ischemia. However, due to the anaerobic metabolism at sites of ischemia, different types and/or amounts of post-translational modifications may occur as a result of ischemia as compared to inflammation only. If desired or necessary, ischemia can be distinguished from inflammation. For instance, an antibody directed to a particular epitope comprising a post-translational modification of a particular protein present only in ischemia or to a particular type of post-translational modification of a particular protein present only in ischemia could be used. Alternatively, a profile of several post-translational modifications could be used to distinguish the two states.

### 1. General Ischemia

In one embodiment ofthe invention, only the presence, absence or degree of ischemia somewhere in an animal is determined. This embodiment is referred to as the general ischemia embodiment to distinguish it from the embodiment of the method of the invention which measures specific types of ischemia. By "specific types of ischemia" is meant ischemia of a particular tissue or organ (*e.g.*, cardiac ischemia). The general ischemia embodiment does not provide a diagnosis of any specific type of ischemia but, in combination with other diagnostic parameters, will be very helpful in diagnosing the specific type of ischemia.

The general ischemia embodiment of the invention can also be used to monitor the ischemia, including determining the effectiveness of treatments of the ischemia. If a particular treatment is not effective in reducing the ischemia, the treatment can be modified. For instance, the dosage of a drug can be increased or another treatment can be substituted for the ineffective treatment. The general ischemia embodiment can also be used to choose the lowest effective dose of a drug and the shortest time during which a drug or treatment needs to be administered. Finally, this embodiment can be used to monitor the effectiveness of new potential treatments of ischemia, as well as known treatments.

To measure general ischemia, all of the post-translationally modified proteins, a subset of the post-translationally-modified proteins, or a single post-translationally modified protein present in a body fluid is(are) measured. Each post-translationally modified protein which is employed in the measurement of general ischemia is referred to herein as a "general marker of ischemia." Preferably, general ischemia is measured by measuring one or more post-translationally modified proteins which are constituents of a body fluid, and these proteins are the preferred general markers of ischemia. Preferably, the body fluid is blood since it comes into contact, or communicates with, essentially all areas of an animal's body. The blood sample is preferably serum or plasma. The most preferred general marker of ischemia is post-translationally modified albumin, and the preferred post-translational modifications are phosphorylation and cysteinylation.

### 2. Diagnosis And Monitoring Of Specific Types Of Ischemia

The present invention can also be used to diagnose and/or monitor specific types of ischemia. In particular, ischemia in a particular organ or tissue can be diagnosed and/or monitored. This is accomplished by measuring the amount(s) of one or more post-translationallymodified tissue-specific or organ-specific proteins present in one or more body fluids.

The following are some examples of organ-specific and tissue-specific proteins suitable for measuring ischemia of particular organs and tissues:
15. For myocardial infarction - troponin I, troponin T, creatinine phosphokinase (CPK), its MB isoenzyme (CPKMB), and myoglobin.
16. For cerebral ischemia - an S100 protein, such as S100B.
17. For bowel ischemia - albumin.
18. For placental ischemia - albumin or pregnancy-associated proteins such as β-human chorionic gonadotropin, α-fetoprotein, pregnancy-associated protein 1A, erythropoietin and angiotensin.

It may be possible to make a definitive diagnosis of a specific type of ischemia based only on the presence, absence and/or level of one or more post-translationally modified organ-specific or tissue-specific proteins in one or more body fluids. However, it may be necessary or desirable to also consider other diagnostic parameters to make the diagnosis. For instance, the use of additional diagnostic parameters may improve the sensitivity and/or specificity of, and/or improve the confidence in, the diagnosis.

This embodiment of the invention can also be used to monitor the specific type of ischemia, including determining the effectiveness of treatments of the ischemia. If a particular treatment is not effective in reducing the ischemia, the treatment can be modified. For instance, the dosage of a drug can be increased or another treatment can be substituted for the ineffective treatment. This embodiment can also be used to choose the lowest effective dose of a drug and the shortest time during which a drug or treatment needs to be administered. Finally, this embodiment can be used to monitor the effectiveness of new potential treatments of specific types of ischemia, as well as known treatments.

### 3. Early Diagnosis Of Cardiac Ischemia

The present invention has been found to be particularly useful for the early diagnosis of cardiac ischemia. By "early diagnosis" is meant ascertaining the presence or absence of cardiac ischemia during the first few hours (less than 24 hours, especially less than 12 hours) following the onset of symptoms indicative of cardiac ischemia, such as chest pain, shortness of breath and pain or tingling in the left arm. The standard diagnostic tests for cardiac ischemia are often inconclusive during this time frame, and reliable early biomarkers do not exist. The present invention provides a diagnostic assay of high sensitivity and specificity, including during the first few hours following the onset of symptoms of cardiac ischemia, and should, therefore, be of great utility in making an early diagnosis of cardiac ischemia.

### 4. No Need To Denature The Post-Translationally Modified Proteins

A method of detecting certain post-translationallymodified tissue-specific and organ-specific proteins present in the serum of patients as a result of cell damage due to, *e.g.*, cardiac ischemia, has been described. See PCT application WO 02/16947 and Labugger et al., Circulation, 102:1221-1226 (2000). In particular, it was reported that phosphorylated troponin I and several degraded forms of troponin I were found in the serum of patients suffering from acute myocardial infarctions using this method. However, it was necessary to denature the proteins in order to be able to detect the phosphorylated troponin I, the degraded forms oftroponin I and other tissue-specific and organ-specific proteins which are markers of cell damage.

It is quite surprising then, that using the methods of the present invention, it is possible to accurately quantitate organ-specific and tissue-specific post-translationally modified proteins without the need to denature them. For instance, a quantitative assay for phosphorylated troponin I using native (*i.e.*, not denatured) troponin I has been developed which is able to diagnose cardiac ischemia with high specificity and sensitivity (see Example 4). Of course, the methods of the present invention are faster, simpler and cheaper than the method described in PCT application WO 02/16947 and Labugger et al., Circulation, 102:1221-1226 (2000), since the steps and reagents required to denature proteins are omitted.

### 5. Diagnosis And Monitoring Of Ischemia By Measuring Phosphorylated Proteins

In a particularly preferred embodiment of the invention, ischemia is diagnosed and/or monitored by quantitating the level of one or more phosphorylated protein(s). It is believed that the phosphorylation of protein(s) during ischemia occurs as a result of:
(i) inflammation, through the action of specific kinases; and
(ii nonspecific substrate level phosphorylation, which occurs as a result of anaerobic metabolism in and near ischemic areas of a tissue or organ.

The substrate level phosphorylation of protein(s) in ischemic areas can result in a large amount of phorphorylation of the protein(s), producing a naturally amplified signal and a particularly robust assay for ischemia.

Thus, in a preferred embodiment of the present invention, ischemia is diagnosed and/or monitored by determining the quantity of one or more phosphorylated proteins present in a body fluid. If the quantity of the phosphorylated protein(s) is significantly increased as compared to the level in the same fluid from normal controls, then the patient is suffering from ischemia. The protein(s) can be one(s) which has(have) been phosphorylated initially at one or more sites or one(s) whose amount of phosphorylation has been increased as a result of the increased kinase activity caused by the inflammation and the substrate phosphorylation caused by the ischemia.

All of the phosphorylated proteins present in the body fluid, a single phosphorylated protein present in the body fluid, or some subset of phosphorylated proteins present in the body fluid can be measured to diagnose and/or monitor general ischemia. One or more phosphorylated organ-specific or tissue-specific proteins present in one or more body fluids can be measured to diagnose and/or monitor specific types of ischemia. Preferably, one or more organ-specific or tissue-specific protein in one or more body fluids is quantitated.

Many methods can be used to quantitate the phosphorylated proteins (see above). Preferably, the phosphorylated proteins present in the body fluid are quantitated by a binding-partner assay, most preferably by an immunoassay (see above).

### 6. Diagnosis And Monitoring Of Ischemia By Measuring Cysteinylated Proteins

In another preferred embodiment of the invention, ischemia is diagnosed by quantitating the level of one or more cysteinylated proteins present in a body fluid. Cysteinylated proteins can be measured by one or more of the methods described above. Preferably, the cysteinylated proteins are measured by a binding-partner assay, more preferably an immunoassay, most preferably an enzyme immunoassay, using appropriate antibodies (*e.g,* antibodies specific for the cysteinylated protein(s) (epitopes which are cysteinylated and/or epitopes which are not cysteinylated)).

It is believed that the levels of cysteinylated proteins are increased in ischemia for the following reasons. First, cystathionine β-synthetase (CBS) is present in many organs and tissues, includingbrain, liver, pancreas, kidney and placenta. It is also present in fetal tissues. Ischemia in these organs and tissues, or portions thereof, or in fetal tissues results in the inhibition of CBS, leading to the accumulation of homocysteine, which then enters non-ischemic areas and the bloodstream. This homocysteine is processed by uninhibited CBS in non-ischemic areas (*e.g.*, in the blood andnon-ischemic areas ofischemic organs and tissues) into cystathionine and cysteine through the transulphuration pathway (see Figure 5) which cysteinylates proteins. Thus, the cysteinylated proteins can include circulating proteins, such as albumin, which are general markers of ischemia. The cysteinylated proteins can also include proteins produced in non-ischemic areas of an ischemic organ or tissue or of a fetus which are, therefore, useful for the diagnosis and monitoring of specific types of ischemia. The latter type of cysteinylated proteins can include cysteinylated brain proteins (such as S100 proteins) and cysteinylated placental and fetal proteins (such as β-human chorionic gonadotropin, α-fetoprotein, pregnancy-associated protein 1A, erythropoietin and angiotensin).

The levels of cysteinylated proteins may be increased during ischemia by other mechanisms. For instance, Cu(II) levels are increased in ischemia, both in the bloodstream and in organs and tissues, so cysteinylation of proteins should be increased during ischemia (see Figure 5). Finally, the level of homocysteine in the blood is generally elevated in those at risk of suffering an ischemic event, and this may also give rise to higher levels of cysteinylation of blood proteins (see Figure 5).

### 7. Ischemia Panels

In another embodiment of the invention, two or more post-translationally modified proteins are quantitated to improve the diagnosis and/or monitoring of ischemia and/or to provide a more complete profile of the ischemia. For instance, two or more general markers of ischemia in one or more body fluids could be measured to improve the diagnosis and/or monitoring of general ischemia. Similarly, the quantities of two or more organ-specific or tissue-specific proteins in one or more body fluids could be determined to improve the diagnosis and/or monitoring of a specific type of ischemia and/or to determine if there is more than one specific type of ischemia present in the animal. Finally, one or more general markers of ischemia in one or more body fluids and one or more organ-specific or tissue-specific proteins in one or more body fluids could be measured to determine if ischemia is present in the animal (general ischemia determination) and, if ischemia is present, the location(s) of the ischemia.

The quantitation of the post-translationally modified proteins can be accomplished by any of the methods described above. For instance, mass spectrometry could provide a profile of the ischemia by identifying and quantitating the post-translationally modified proteins in one or more body fluids. Preferably, however, a collection of binding partners specific for the post-translationally modified proteins of interest will be provided. Such a collection may be a collection of test tubes, each holding a binding partner, or a microtiter plate, each well of which contains a binding partner, or an array of binding partners on a substrate. The collection of binding partners is then employed to prepare a profile of the ischemia.

### 8. Diagnosis And Monitoring Of Ischemia By Use Of a Combination Of A Level Of A Post-Translationally Modified Protein And Another Diagnostic Parameters

To diagnosis ischemia with higher sensitivity and/or specificity and/or with improved confidence, it may be desirable to use the presence, absence or levels of post translationally modified proteins in combination with other diagnostic parameters. For instance, a medical history and other laboratory and clinical findings may be helpful or necessary to diagnose or rule out ischemia with confidence.

As one example, it may be desirable to use a determination of the level of phosphorylated troponin I in combination with a standard (non-phosphorylated) troponin I or CK-MB level for diagnosis of myocardial ischemia. As another example, it may be desirable to use a determination of the level of phosphorylated albumin or cysteinylated albumin in combination with a standard (non-phosphorylated) troponin I or CK-MB level for diagnosis of myocardial ischemia. Similarly, it may be desirable to use a determination of the level of phosphorylated albumin or cysteinylated albumin in combination with a standard marker of brain ischemia for the diagnosis of that condition.

### E. Improved Method Of Diagnosing Appendicitis

The present invention further provides an improved method of diagnosing appendicitis in an animal, preferably a human. The first step of the method is to determine if the quantity of orthohydroxyhippuric acid (OHHA) in a body fluid, preferably urine, is significantly elevated compared to its level in the same body fluid from normal animals.

This step can be performed as described in U.S. Patent No. 5,470,750 and Example 9 below. The complete disclosure of U.S. Patent No. 5,470,750 is incorporated herein by reference.

U.S. Patent No. 5,470,750 describes several methods of measuring OHHA. These methods include color-producing chemical reactions followed by detection/measurement of the color using the naked eye or by fluorescence, ultraviolet or visible spectroscopy ("colorimetric assays"). The methods disclosed in U.S. Patent No. 5,470,750 for measuring OHHA further include mass spectrometry, various types of chromatography (including thin layer chromatography, high pressure liquid chromatography and gas chromatography), nuclear magnetic resonance, enzymatic reactions, and immunoassays.

A preferred embodiment of U.S. PatentNo. 5,470,750 is a colorimetric screening test. To perform this screening test, urine obtained from an animal to be diagnosed is added to a color-producing reagent. Preferably, the color-producing reagent is ferric ions bonded to silica or another support material (such as Celite®, clays, alumina and/or fire brick). The ferric-bonded silica can be prepared as follows. Forty grams of ferric nitrate hexahydrate is dissolved in 40 ml of 25% hydrochloric acid and sonicated for 15 minutes. Then, 100 grams of silica is slowly stirred into the ferric nitrate solution until a free flowing powder is obtained. To perform the screening test, urine is added to the ferric-bonded silica powder and, after mixing vigorously, is incubated to allow the color to develop. A brown, red, or violet color in the top layer of liquid indicates a quantity of OHHA in the urine above a threshold value indicative of appendicitis. If the color of the top layer of liquid is normal urine light yellow or pink, either OHHA is not present or is present below the threshold level indicative of appendicitis. The color is best judged by comparison to either a color comparison chart or to the colors produced by standards run at the same time as the test sample(s). The color comparison chart may be prepared by using known amounts of pure OHHA in normal urine in the screening test and photographing the resultant colors. The standards will similarly comprise one or more known amounts of pure OHHA that can be diluted in normal urine and run in the screening test at the same time as the test sample(s). The standards will include at least an amount of pure OHHA that is the amount of OHHA indicative of appendicitis, and will preferably include several different amounts of pure OHHA that will give a range of colors indicative of the absence of appendicitis (negative controls) and of the presence of appendicitis (positive controls).

U.S. PatentNo. 5,470,750 also describes a thin layer chromatography (TLC) method that can be used to confirm a positive result (*i.e.*, a color indicative of appendicitis) in the colorimetric screening test. This method involves the extraction of the OHHA from the urine sample. This can be accomplished by acidifying the urine (*e.g.*, with citric acid, hydrochloric acid or an ion exchange resin), passing the acidified urine through a column filled with C18 bonded silica, and eluting the OHHA with sodium acetate buffer (0.05 M, pH 6.0) or methanol. The extracted OHHA is then subjected to TLC, preferably using thin layer silica plates supported on glass, metal or plastic backing without any fluorescent indicator. To perform the TLC, the extracted OHHA is spotted onto a plate, and the plate is developed using an appropriate mobile phase (for instance, for methanol-extracted OHHA, the mobile phase is 1 part glacial acetic acid and 99 parts ethyl acetate). The OHHA can be detection using either a 250-300 nm ultraviolet light (which produces a blue fluorescing spot if OHHA is present above a threshold level indicative of appendicitis) or by spraying or dipping the plate in a solution of ferric nitrate in methanol (in which case, a red spot is produced if OHHA is present above a threshold level indicative of appendicitis). The plates are always run with at least one standard of pure OHHA at the threshold concentration which is indicative of appendicitis.

A kit for the colorimetric determination of OHHA is available from DMI BioSciences, Inc., Englewood, Colorado (AppyTest® assay kit). This kit, which is covered byU.S. PatentNo. 5,470,750, includes apparatus, reagents and other materials for performing both the screening test and the TLC assay described above.

Most preferably, however, the quantity of OHHA present in a biological fluid will be measured bya binding partner assay using a binding partner specific for OHHA. The binding partner assay may be any of those described above for measuring post-translationally modified proteins and is preferably an immunoassay, most preferably an enzyme immunoassay. Also, the binding partner may be any of those described above and is preferably an antibody or aptamer specific for OHHA. Since OHHA is a small molecule, it will have to be attached to an immunogenic carrier to prepare antibodies. Suitable immunogenic carriers (such as Keyhole Limpethemocyanin (KLH), albumins and other high molecular weight proteins and polypeptides) and methods of coupling small molecules to them are well known in the art. See, e.g., U.S. Patent No. 5,484,735 and the references cited therein. The binding partner assays are preferred since they will give a quantitative measure of the amount of OHHA present in a body fluid and will allow for more precise determinations of whether the quantity of OHHA present in the body fluid is elevated compared to its level in the same body fluid from normal animals.

As noted in the Background, despite its limitations, the total leukocyte count continues to be the most widely used laboratory test to assist in the evaluation of clinically suspected appendicitis. Although the colorimetric screening test for OHHA described above has significantly higher specificity than elevated leukocyte counts (80.6% versus 67.9%, p<0.01; see Example 9 below) for the diagnosis of appendicitis, an assay that provided greater specificity and diagnostic accuracy than either total leukocyte counts or OHHA levels alone would be desirable. The present invention provides such an assay for diagnosing appendicitis.

In particular, it has been found that the specificity and accuracy of the OHHA assay can be improved by also measuring the quantity of a marker of general inflammation present in a body fluid from the animal being diagnosed. The body fluid may be same body fluid used for the measurement of the OHHA or maybe a second, different body fluid. Preferably, the body fluid for the measurement of the marker of general inflammation is urine, plasma or serum.

The marker of general inflammation may be a known marker of general inflammation, such as the total leukocyte count, neutrophil count, neutrophil band count, or C-reactive protein level. Methods of measuring these markers are well known and routine in clinical laboratories. Also, the normal ranges and the values which are indicative of inflammation are also well known or can be determined empirically. A preferred marker of general inflammation is total leukocyte count. The normal range for total leukocyte counts in adult humans is 5-10 x 10⁹/liter, and a count of 12 x 10⁹/liter or greater indicates the presence of inflammation. The normal range for children varies depending on age, as does the count which indicates inflammation (see Example 9 below).

Another preferred marker of general inflammation is an inflammatory cytokine, such as interleukin 8 (IL-8). The quantity of the cytokine in a body fluid can be measured as described above for the post-translationally modified proteins. Preferably, the cytokine is measured using a binding partner assay, most preferably an immunoassay. The preferred binding partners are antibodies and aptamers specific for the cytokine. It can be determined whether the level of the cytokine in the body fluid of the animal being diagnosed is significantly elevated compared to its level in the same body fluid from normal controls using standard statistical methods well known in the art, such as those described above.

In particular, elevated levels ofIL-8 have been found in the urine ofpatients suffering from inflammation and inflammatory diseases, including appendicitis. See PCT application WO 01/11371. The quantity of IL-8 in urine maybe measured as described above for the post-translationally modified proteins. Preferably, the IL-8 is measured using a binding partner assay. Preferably the binding partner assay is an immunoassay, most preferably an enzyme immunoassay. Preferably, the binding partner is an antibody or aptamer specific for IL-8. It is reported in PCT application WO 01/11371 that normal controls (twenty controls) had 3-43 pg/ml of IL-8 in their urine as measured by an ELISA assay. By comparison, eight appendicitis patients had 44-430 pg/ml of IL-8. No statistical analysis of these data was reported, but at least five of the eight appendicitis patients had substantially elevated levels of IL-8 (74-430 pg/ml) as compared to the normal controls.

A third and particularly preferred marker of general inflammation is a post-translationally modified protein which is a protein marker of general inflammation. These post-translationally modified proteins and their measurement, including how to determine if the proteins are significantly elevated compared their levels in the same body fluid(s) from normal animals, are described above. Preferred are one or more post-translationally modified proteins which are constituents of a body fluid, preferably blood. The protein constituents of blood include albumin and immunoglobulins. The blood sample is preferably serum or plasma. The most preferred protein marker of general inflammation is post-translationally modified albumin, and the preferred post-translational modifications are phosphorylation and cysteinylation.

However, acetylated proteins should not be used as the post-translationally modified protein in cases where a patient has ingested aspirin, since aspirin (acetylsalicylic acid) acetylates proteins. The level of an acetylated protein will not accurately reflect the presence of inflammation in such cases.

The use of a combination of an OHHA assay and an assay for a marker of general inflammation improves the specificity and diagnostic accuracy as compared to use of the OHHA assay alone or use of the assay for a marker of general inflammation alone. In particular, it has been found that concordant results of the OHHA assay and an assay for a marker of general inflammation provide substantiallyimproved specificity and accuracy (see, *e.g.*, Example 9). By concordant results, it is meant that both tests are positive or both tests are negative. For instance, if the quantity of OHHA is significantly elevated as compared to its level in normal controls and the quantity of the marker of general inflammation is also significantly altered as compared to its level in normal controls, then appendicitis is indicated. Similarly, if the quantity of OHHA is not significantly elevated and the quantity of the marker of general inflammation is not significantly altered, then appendicitis is not indicated. If one test is positive and the other test is negative, then further information is needed to make a diagnosis. For instance, the two tests may be repeated after a period of time (*e.g.*, 8-12 hours).

Also, when using a combination of an OHHA assay and an assay for a marker of general inflammation, it is possible to use only the colorimetric screening test for OHHA described above (see, *e.g.*, Example 9). The use of the time-consuming and complicated TLC confirmatory test can be eliminated. Thus, in a preferred embodiment, the invention provides an easy and rapid method of diagnosing appendicitis by using the colorimetric screening test for OHHA in combination with an assay for a marker of general inflammation.

In another preferred embodiment, the invention provides a combination of a binding partner assay for OHHA and a binding partner assay for a marker of general inflammation. Preferably, one or both of the binding partner assays are immunoassays.

### F. Kits

The invention also provides kits for determining the quantities of post-translationally modified proteins and kits for diagnosing appendicitis. The kits may be formatted for use in a diagnostic apparatus (*e.g.*, an automated analyzer) or can be self-contained (*e.g.*, for a point-of-care diagnostic).

Kits for determining the quantities of post-translationally modified proteins will comprise one or more containers holding reagents useful for performing the assays, including, particularly, containers holding binding partners useful for quantitating post-translationally modified proteins. Suitable containers for the reagents of the kit include bottles, vials, test tubes and microtiter plates. Also, reagents (*e.g.*, binding partners) can be incorporated into or onto substrates, test strips (made of, *e.g.*, filter paper, glass, metal, plastics or gels) and other devices suitable for performing binding-partner assays, including arrays of binding partners on substrates. Instructions for performing one or more assays for quantitating a post-translationally modified protein will be provided with the kits (*e.g.*, the instructions can be provided in the same package holding some or all of the reagents or can be provided in separate documentation). The kit may also contain other materials which are known in the art and which may be desirable from a commercial and user standpoint, such as buffers, enzyme substrates, diluents, standards, etc. Finally, the kit may include containers, such as empty containers for performing the assay, for collecting, diluting and/or measuring a body fluid, and/or for diluting reagents, etc.

Kits for diagnosing appendicitis will comprise one or more containers holding reagents useful for diagnosing appendicitis, including, particularly, reagents for assaying for OHHA and/or reagents for assaying for a marker of general inflammation. The kits may be two-part kits, each part providing the reagents and other materials for performing one of the assays. Alternatively, two different kits may be provided. Instructions for performing each of assays will be provided with the kits (*e.g.*, the instructions can be provided in the same package holding a two-part kit, can be provided in each of the packages holding the separate kits, or can be provided in separate documentation). Suitable containers for the reagents include bottles, vials, test tubes, and microtiter plates. Also, reagents (*e.g.*, binding partners) can be incorporated into or onto substrates, test strips (made of, *e.g.*, filter paper, glass, metal, plastics or gels) and other devices suitable for performing binding-partner assays, including arrays of binding partners on substrates. The kit(s) may also contain other materials which are known in the art and which may be desirable from a commercial and user standpoint, such as buffers, enzyme substrates, diluents, standards, etc. Finally, the kit may include containers, such as empty containers for performing the assay, for collecting, diluting and/or measuring a body fluid, and/or for diluting reagents, etc.

For performing an OHHA assay, the kit may comprise a container holding a color-producing material (*i.e.*, a material capable of undergoing a color-producing reaction when contacted with OHHA). Preferably, the color-producing material comprises ferric ions. Most preferably, the color-producing material is ferric ions bonded to silica. Such a kit may further comprise a container for collection of a body fluid (such as a syringe or a plastic or paper cup), an instrument for measuring the body fluid (such as a dropper, a pipette or a micropipette) and either a color comparison chart or containers holding standards comprising known amounts of OHHA.

Alternatively, a kit for performing an OHHA assay may comprise a container holding a binding partner specific for OHHA. Such a kit may further comprise a container for collection of a body fluid (such as a syringe or a plastic or paper cup), an instrument for measuring a volume of the body fluid (such as a dropper, a pipette or a micropipette) and containers holding buffers, enzymes, enzyme substrates and other reagents, including standards comprising known amounts of OHHA.

Kits and reagents for assaying for markers of general inflammation such as total leukocyte count, neutrophil count, neutrophil band count and C-reactive protein are well known. Typically, such kits and reagents will already be available in clinical laboratories, but may also be provided, particularly as part of a two-part kit.

When the marker of general inflammation is a cytokine or a post-translationally modified protein, the kit may comprise a container holding a binding partner specific for the cytokine or post-translationally modified protein. Such a kit may further comprise a container for collection of a body fluid (such as a syringe or a plastic or paper cup), an instrument for measuring a volume of the body fluid (such as a dropper, a pipette or a micropipette) and containers holding buffers, enzymes, enzyme substrtaes and other reagents, including standards comprising known amounts of the cytokine or post-translationally modified protein.

### EXAMPLES

The following Examples are intended to illustrate embodiments of the invention and are not intended to limit the invention.

### EXAMPLE 1: Phosphorylated Albumin Is A Marker Of Inflammation And Ischemia

Mouse monoclonal antibody to human albumin (clone HSA-11, Sigma, St. Louis, MO, catalog number A-6684) was diluted to 1 µg/ml in phosphate buffer saline (PBS), pH 7.4, and 100 µl of this solution were added to each well of ELISA strip wells (Nunc, F16 Maxisorp strips, catalog number 469914). After incubation overnight at room temperature, the antibody solution was aspirated, and the strips were blotted dry on a paper towel.

Next, 200 µl of PBS containing 4% bovine serum albumin (BSA) were added to each well. After incubation for 18-24 hours at room temperature, the BSA solution was aspirated, and the strips were blotted dry on a paper towel.

Then, 50 µl of each patient's serum or plasma were added to the wells in duplicate. As a control, 50 µl of PBS containing 4% bovine serum albumin (BSA) were added to duplicate wells to determine background. After incubation for 1 hour at room temperature with gentle shaking, the samples were aspirated, and the wells were washed 4 times with wash buffer (50 mM Tris, pH 7.9-8.1,0.2% Tween 20) (filling the well to the top constituted one wash).

Next, 1 µg/ml rabbit polyclonal anti-phosphoprotein antibody (Zymed Laboratories, catalog number 61-8300) in PBS containing 4% BSA was mixed with a 1:10,000 dilution in PBS containing 4% BSA of goat anti-rabbit IgG conjugated to horseradish peroxidase (HRP; Pierce, catalog number 314600), and 100 µl of this mixture were added to each well. After incubation for 1 hour with gentle shaking, the wells were washed 4 times with wash buffer as described above, and 100 µl TMB substrate (Pierce, catalog number N-301) were added to each well. After incubation for 30 minutes, the reaction was stopped by adding 100 µl stop solution (0.18 M H₂SO₄).

The optical density (OD) of each well was read at 450 nm and 530 nm on a spectrophotometer plate reader. The OD at 530 nm was subtracted from the OD at 450 nm, and the background (OD at 530 nm minus OD at 450 nm) was subtracted from this number. The results are presented in Table 1 below.

**TABLE1**

| **SAMPLE** | **CORRECTED OD (OD 450 minus OD 530 minus background¹)** | **STANDARD DEVIATION** |
|---|---|---|
| Normal human serum | -0.006 | 0.0007 |
| Normal human plasma | -0.002 | 0.0095 |
| R256 (ischemic plasma) | 0.374² | 0.0255 |
| R258 (ischemic serum) | 0.2995² | 0.0057 |
| R258 (ischemic plasma) | 0.348² | 0.018 |
| R260 (ischemic plasma) | 0.054² | 0.0092 |
| R261 (ischemic plasma) | 0.043² | 0.0021 |
| R262 (ischemic plasma) | 0.055² | 0.005 |
| R259 (ARDS³ plasma) | 0.236 | 0.0091 |
| R197 (sepsis plasma) | -0.0065⁴ | 0.0042 |

| | | |
|---|---|---|
| Background was 0.0695. ²Ethylenediaminetetraacetic acid (EDTA) was used as the anticoagulant for samples R260, R261 and R262. Heparin was used as the anticoagulant for samples R256 and R258. EDTA is known to inhibit alkaline phosphatase and can be used to "freeze" the phosphorylation status of the samples (phosphatase inhibitors reduce or prevent dephosphorylation of phosphorylated proteins and peptides), but it may be inhibiting the HRP in the assay. ³ARDS = adult respiratory distress syndrome. ⁴It was subsequently learned that this patient was being treated with steroids. | | |

### EXAMPLE 2: Phosphorylated Albumin Is A Marker Of Inflammation And Ischemia

The assay described in Example 1 was repeated with additional serum and plasma samples. The results are presented in Table 2 below.

**TABLE 2**

| **SAMPLE** | **CORRECTED OD (OD 450 minus OD 530 minus background)** | **COMMENTS** |
|---|---|---|
| Normal human serum (n=3) | 0.005 ± 0.001 | |
| R251 (AMI⁵) | 0.441 | Three hours of CP⁶ - required PTCA⁷ |
| R252 (unstable angina) | 0.265 | Required CABG⁸ |
| R253 (chest pain) | 0.040 | Non-cardiac after cardiology evaluation |
| R258 (chest pain) | 1.671 | Cardiomyopathy, angina |
| ETT-114 (thallium treadmill⁹) | 0.101 | Positive- required emergency CABG |
| ETT-146 (thallium treadmill) | 0.197 | Positive for ischemia |
| R259 (trauma, ARDS) | 2.457 | Hemorrhagic shock, acidosis |
| R268 (multiple trauma) | 0.112 | Pneumothorax |
| R269 (COPD¹⁰) | 0.038 | On steroids |
| R254 (non-Q¹¹ AMI) | 0.027 | COPD on steroids |

| | | |
|---|---|---|
| ⁵AMI = acute myocardial infarction ⁶CP = chest pain ⁷PTCA = percutaneous angioplasty ⁸CABG = coronary artery bypass graft. ⁹A regular treadmill test with the addition of intravenous injection of thallium (radioactive) and detection of the distribution of radioactivity with a focused camera on the heart. Areas which become ischemic with exercise do not perfuse well and will be seen as low thallium counts compared to other areas and at rest. ¹⁰ COPD = chronic obstructive pulmonary disease. ¹¹ non-Q = no q wave seen on electrocardiogram. | | |

### EXAMPLE 3: Phosphorylated Troponin I Is A Marker Of Cardiac Inflammation And Ischemia

Goat monoclonal antibody to human cardiac troponin I (C-19, Santa Cruz Biotechnology, catalog number sc-8118) was dilutedto 1 µg/ml in phosphate buffered saline (PBS), pH 7.4, and 100 µl of this solution were added to each well of ELISA strip wells (Nunc, F16 Maxisorp strips, catalog number 469914). After incubation overnight at room temperature, the antibody solution was aspirated, and the strips were blotted dry on a paper towel.

Next, 200 µl of PBS containing 4% bovine serum albumin (BSA) were added to each well. After incubation for 18-24 hours at room temperature, the BSA solution was aspirated, and the strips were blotted dry on a paper towel.

Then, 50 µl of each patient's serum or plasma were added to the wells in duplicate. As a control, 50 µl of PBS containing 4% bovine serum albumin (BSA) were added to duplicate wells to determine background. After incubation for 45 minutes at room temperature with gentle shaking, the samples were aspirated, and the wells were washed 4 times with wash buffer (50 mM Tris, pH 7.9-8.1, 0.2% Tween 20) (filling the well to the top constituted one wash).

Next, 1µg/ml rabbit polyclonal anti-phosphoprotein antibody (Zymed Laboratories, catalog number 61-8300) in PBS containing 4% BSA was mixed with a 1:10,000 dilution in PBS containing 4% BSA of goat anti-rabbit IgG conjugated to horseradish peroxidase (HRP; Pierce, catalog number 314600), and 100 µl of this mixture were added to each well. After incubation for 45 minutes with gentle shaking, the wells were washed 4 times with wash buffer as described above, and 100 µl TMB substrate (Pierce, catalog number N-301) were added to each well. After incubation for 30 minutes, the reaction was stopped by adding 100 µl stop solution (0.18 M H₂SO₄).

The optical density (OD) of each well was read at 450 nm and 530 nm on a spectrophotometer plate reader. The OD at 530 nm was subtracted from the OD at 450 nm, and the background (OD at 530 nm minus OD at 450 nm) was subtracted from this number. The results are presented in Table 3 below.

**TABLE 3**

| **SAMPLE** | **CORRECTED OD (OD 450 minus OD 530 minus background)** | **COMMENTS** |
|---|---|---|
| Normal human serum (n=2) | 0.085 ± 0.0002 | |
| R254(non-QAMI) | 0.183 | COPD on steroids |
| R258 (chest pain) | 0.307 | Microvascular ischemia |
| 01-009¹² (before angioplasty) | 0.220 | Severe, multi-vessel disease, required PTCA, Troponin I <0.4 |
| 01-009 (30 minutes after angioplasty) | 0.193 | Troponin I <0.4 |
| 01-009 (6 hours after angioplasty) | 0.181 | Troponin I <0.4 |
| 01-009 (24 hours after angioplasty) | 0.162 | Troponin I 0.5 |
| ETT-114 (thallium treadmill) | 0.350 | Positive - required emergency CABG |
| ETT-146 (thallium treadmill) | 0.135 | Positive for ischemia |
| ETT-157 (thallium treadmill) | 0.173 | Positive for ischemia |
| R259 (trauma, ARDS) | 0.409 | Hemorrhagic shock, ischemia because of the hemorrhage, acidosis |

| | | |
|---|---|---|
| ¹²Patient was a 74-year-old female with multi-vessel disease. Patient had PTCA with a stent of the left anterior descending (LAD) complicated by a side branch occlusion. Patient had chest pain, ventricular dysrhythmias and ST-changes (changes of the st segment of the electrocardiogram) during the balloon inflations. | | |

### EXAMPLE 4: Phosphorylated Troponin I Is A Marker Of Cardiac Inflammation And Ischemia

Each year in the United States, approximately 6-8 million people present to a hospital emergency room (ER) with chest pain or other cardiac symptoms (*e.g*., shortness of breath and pain or tingling in the left arm). Unfortunately, about 2-5% of the 3-4 million that are sent home from the ER are mistakenly diagnosed. Chest pain diagnostic errors are the leading cause of emergency medicine malpractice awards.

Of the other 3-4 million that are hospitalized, about 60-75% do not have cardiac disease. The minimum cost for each hospitalized patient is $3,000-5,000, which means that over 6 billion healthcare dollars are wasted each year because of these unnecessary hospitalizations.

With a non-diagnostic electrocardiogram (ECG), reliable early biomarkers do not exist. Troponin I or troponin T levels are unreliable during the first 6-24 hours after the onset of symptoms due to low sensitivity. CK-MB and myoglobin are not cardiac specific.

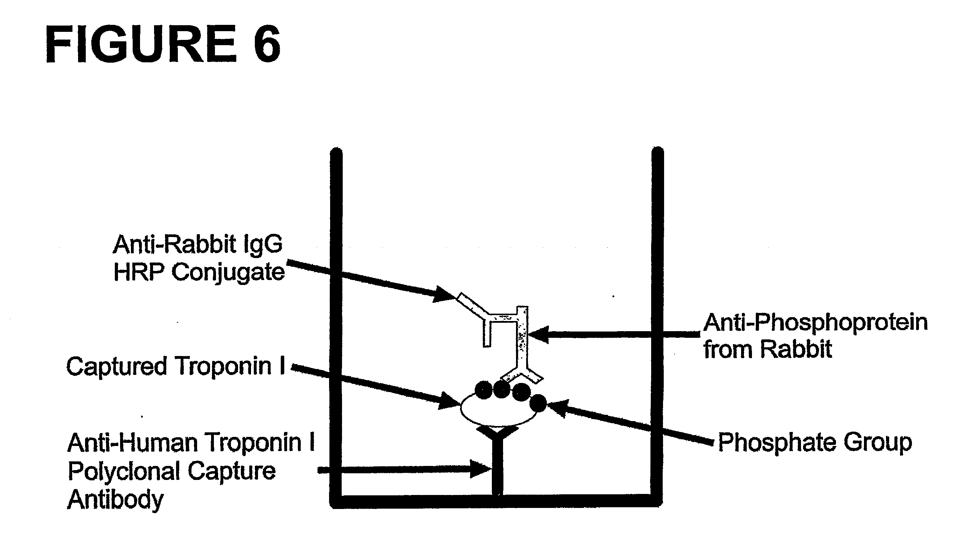

The study population was ER patients presenting with chest pain or symptoms suggestive of acute coronary syndrome (ACS). Normal cardiac troponin I (cTnI) and phosphorylated cardiac troponin I (PO₄-cTnI or PO₄-Trop) were measured in the first blood sample taken from each patient after symptom onset. PO₄-cTnI was measured as described in Example 3, except that a polyclonal antibody specific for human cTnI (Santa Cruz Biotechnology) was used (see Figure 6). The threshold was 0.114 as determined by receiver operating curve (ROC). cTnI was measured using the Dade-Behring Dimension test (threshold - 0.59 by ROC). An ECG was performed on each patient at the time the blood sample was taken (threshold - STEMI, or "high risk" (transient ST elevation of new ST depression > 0.5mm)). ACS was diagnosed by coronary angiography orphysician diagnosis.

Further details concerning the patient population and their outcomes are presented in Table 4.

**TABLE 4**

| **PATIENT CATEGORY** | **NUMBER (%) or YEARS** |
|---|---|
| Total patient samples | 67 (100%) |
| Males | 38 (57%) |
| Females | 29 (43%) |
| Mean age | 59.8 years |
| Hospitalized | 55 (82%) |
| Went home from ER | 12 (18%) |
| Coronary angiography | 33 (49%) |
| Angioplasty [percutaneous angioplasty (PTCA) or percutaneous intervention (PCI)] | 19 (28%) |
| Coronary artery bypass graft (CABG) | 4 (6%) |
| Exercise thallium treadmill | 3 (4%) |
| Healthy controls | 2 (3%) |

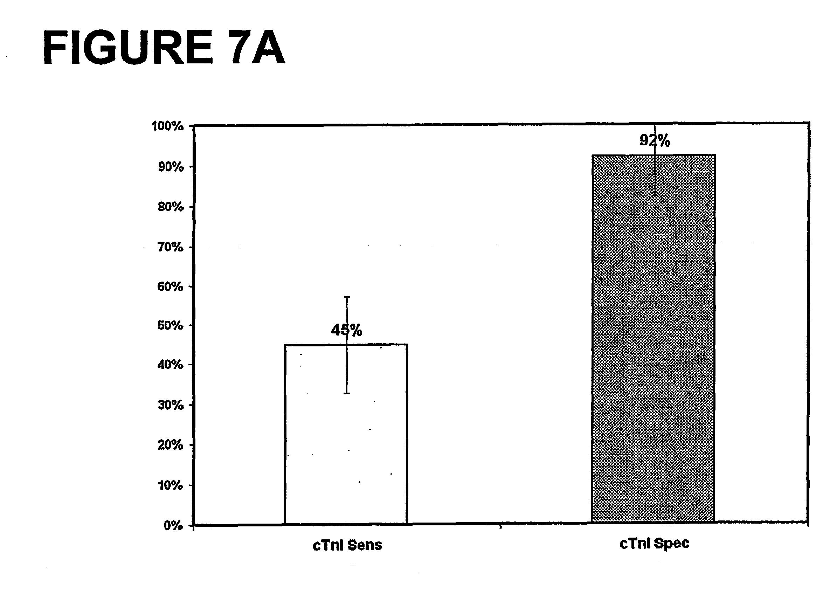

Figures 7A-7S show and compare the sensitivities and specificities of ECG, cTnI, phosphorylated cTnI (PO₄-cTnI or PO₄-Trop) and combinations of these three parameters for the diagnosis and ruling out of ACS. Sensitivity = true positives/(true positives + false negatives). Specificity= true negatives/(true negatives + false positives). In Figures 7A-7S, the following abbreviations have the following meanings: Sens = sensitivity; Spec = specificity; STEMI = ST elevation myocardial infarction; and UA = unstable angina.

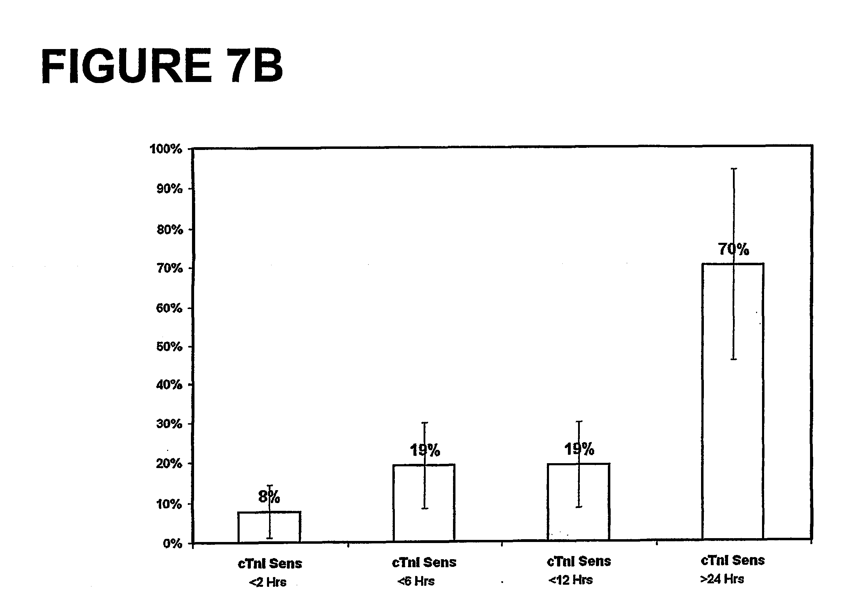

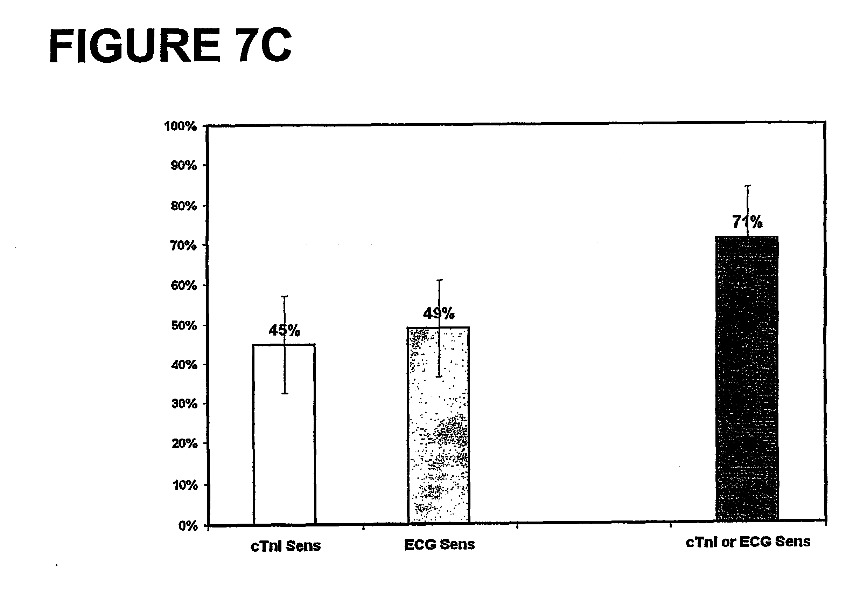

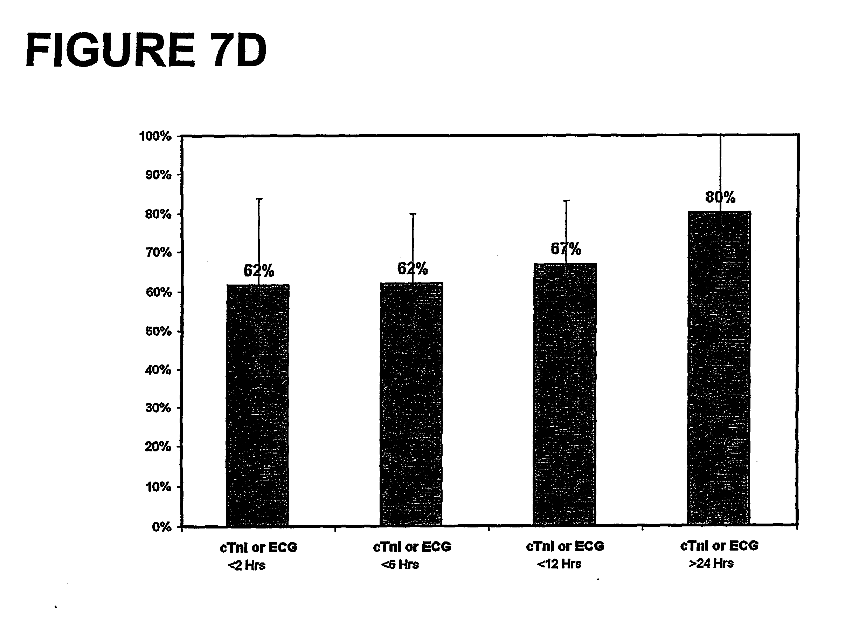

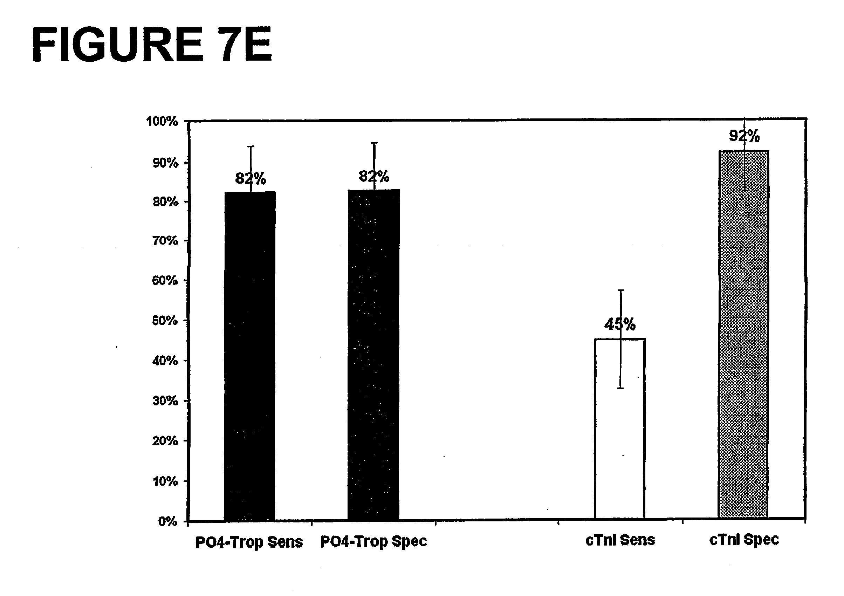

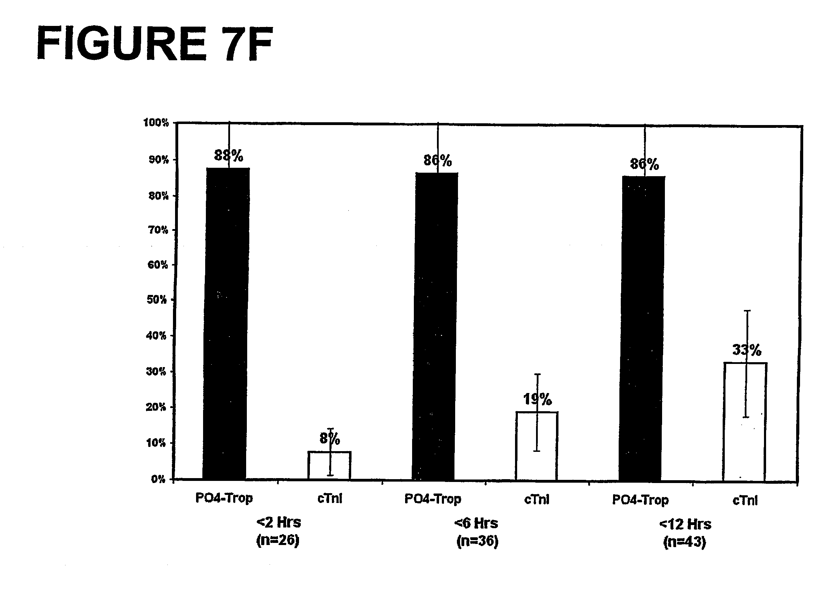

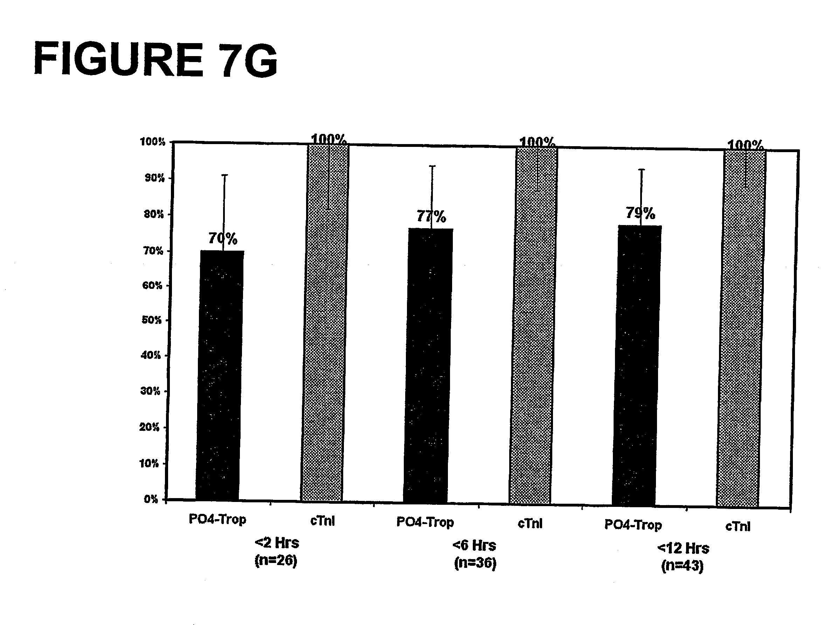

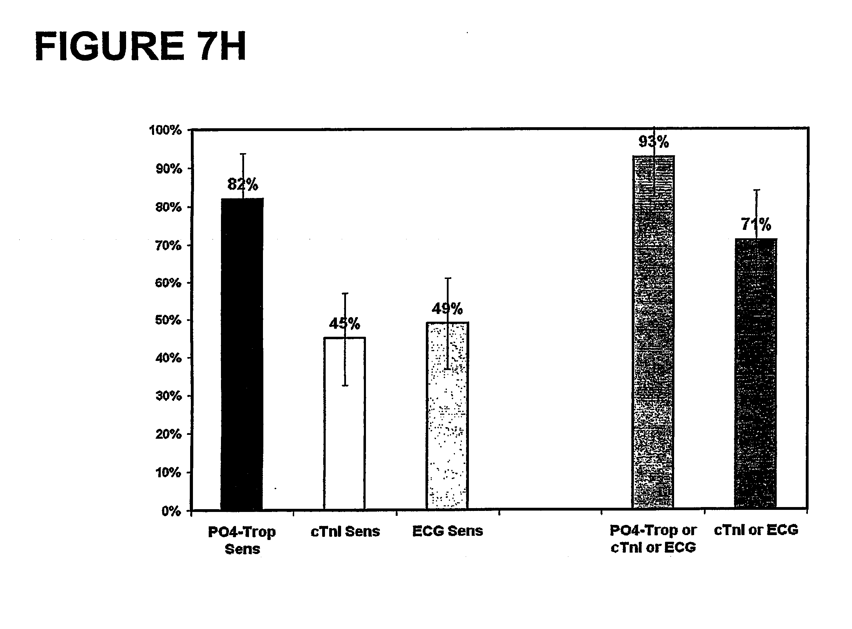

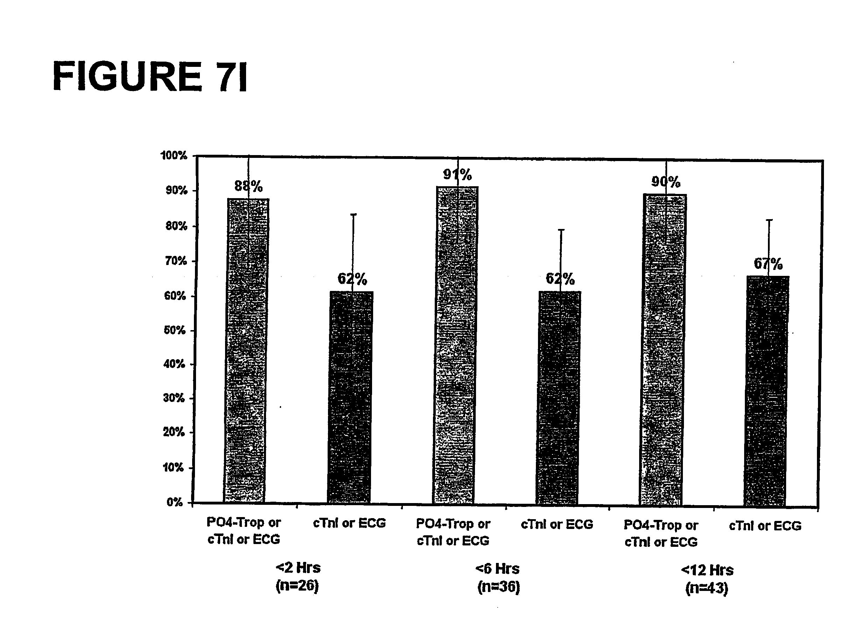

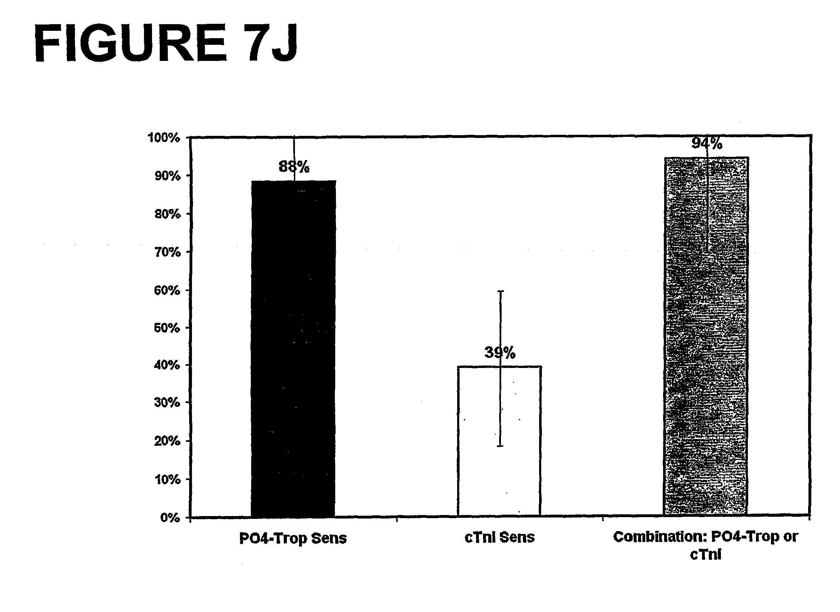

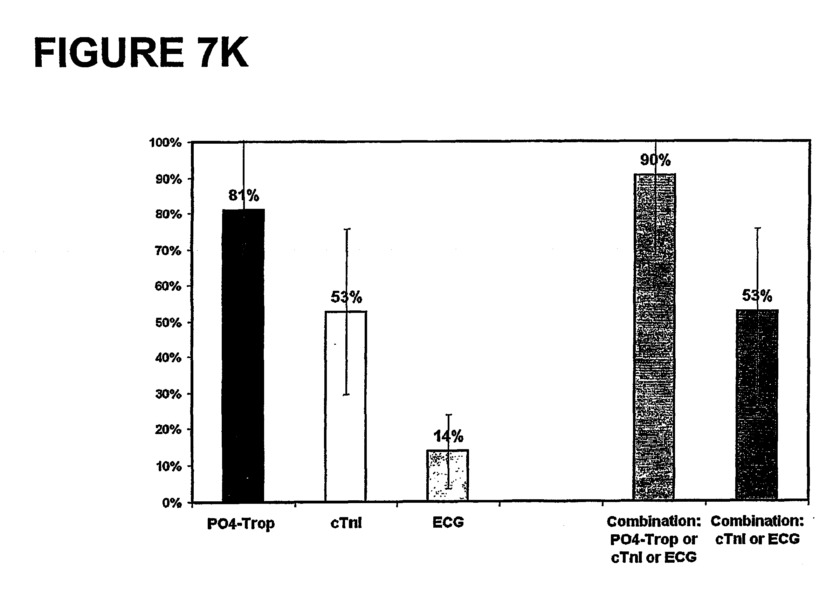

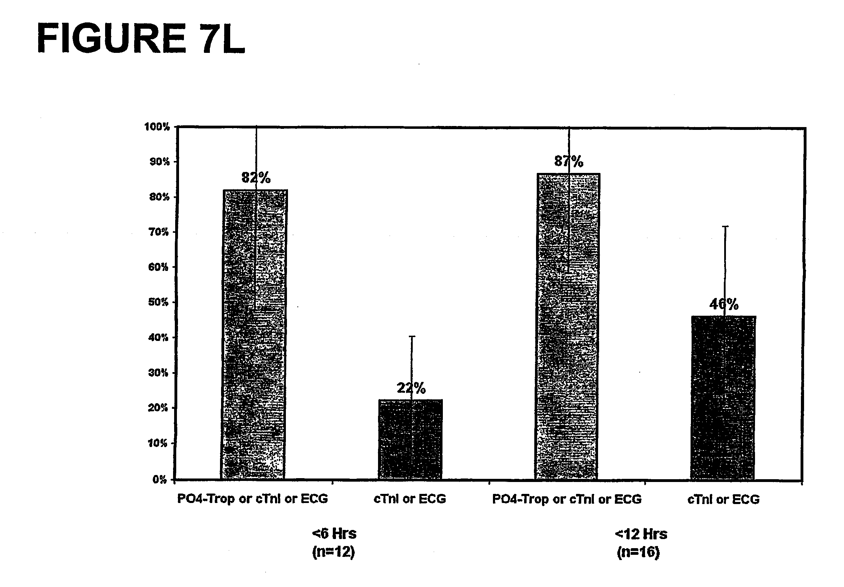

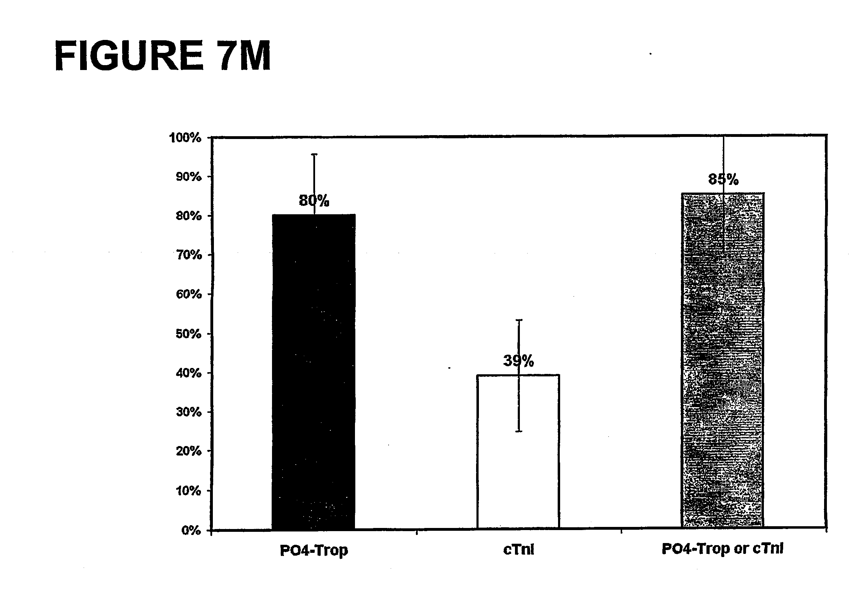

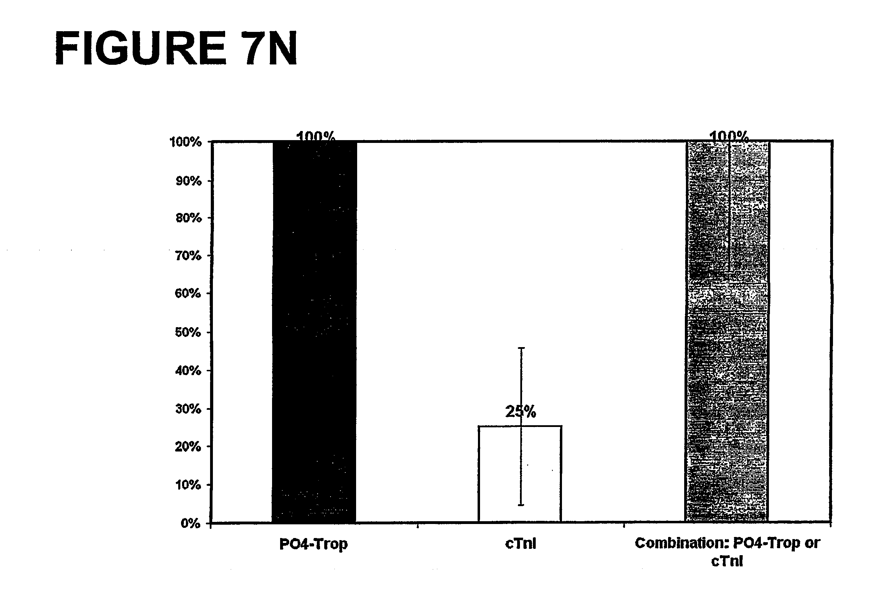

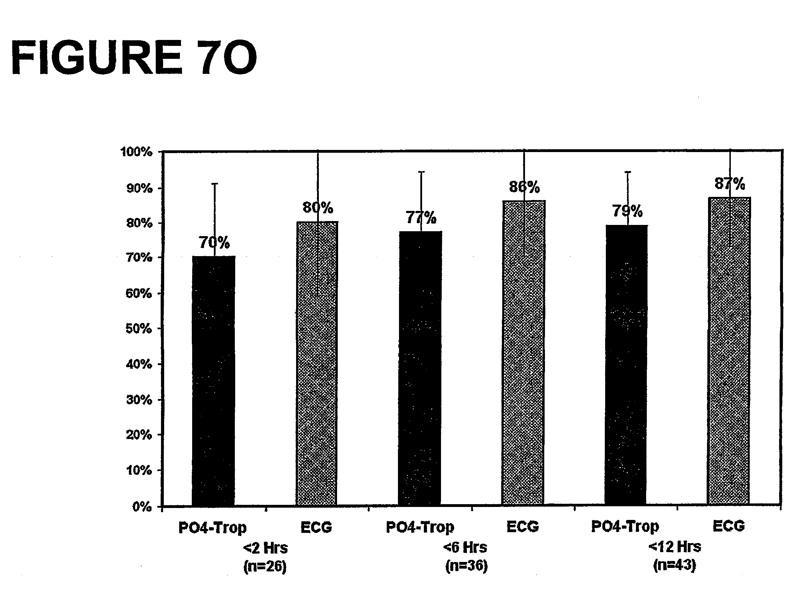

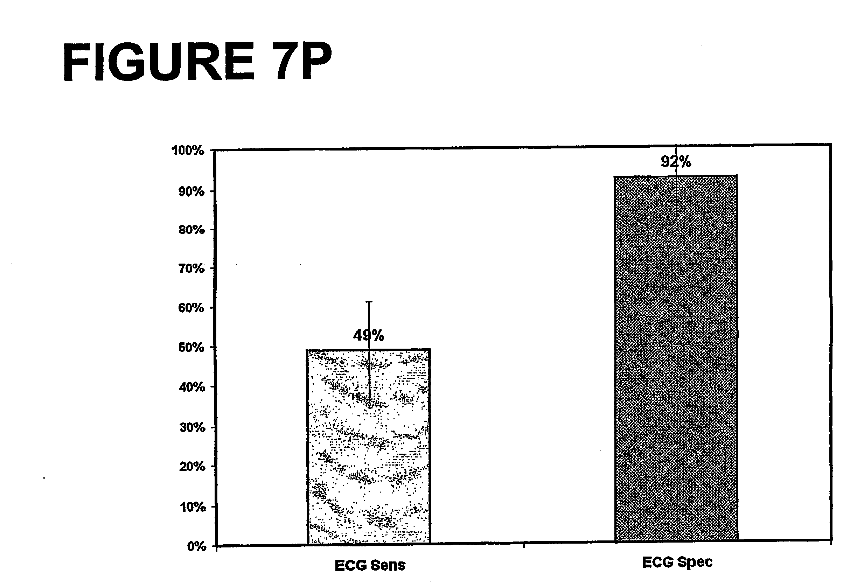

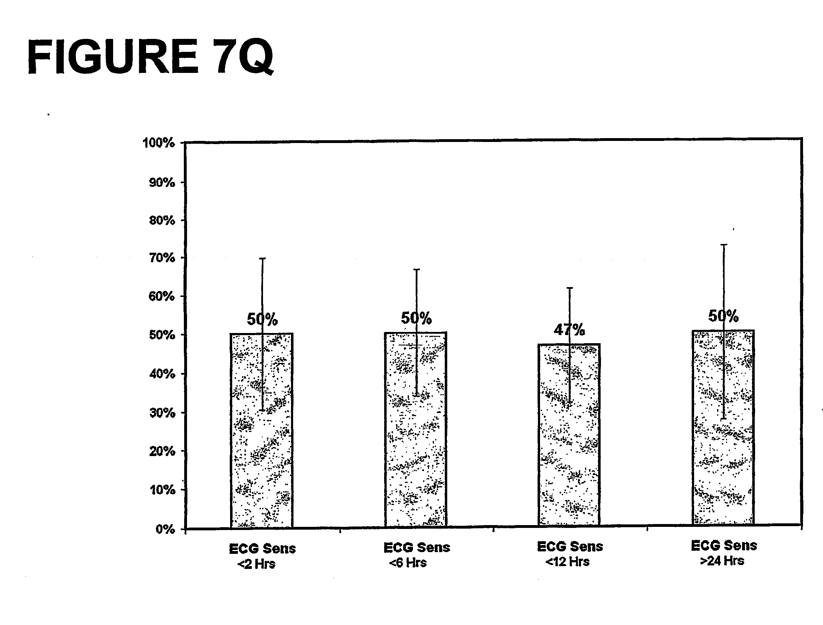

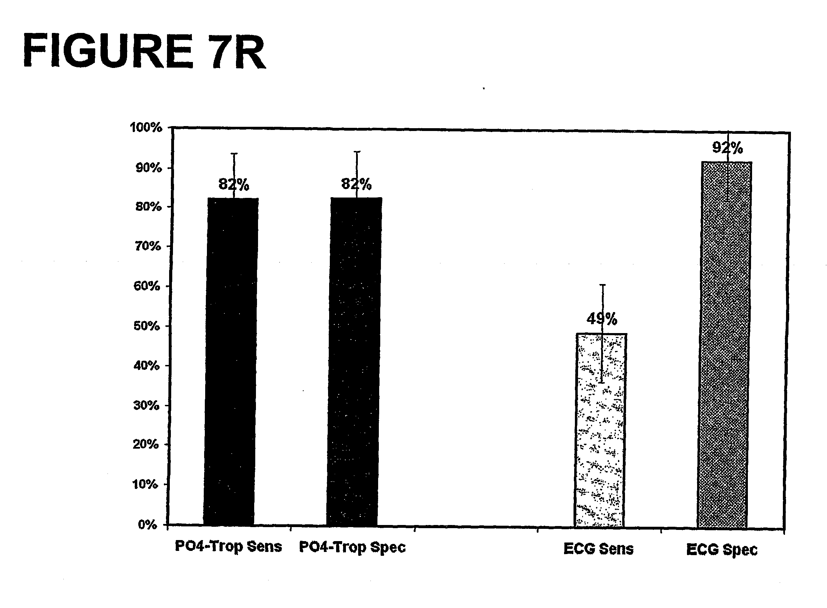

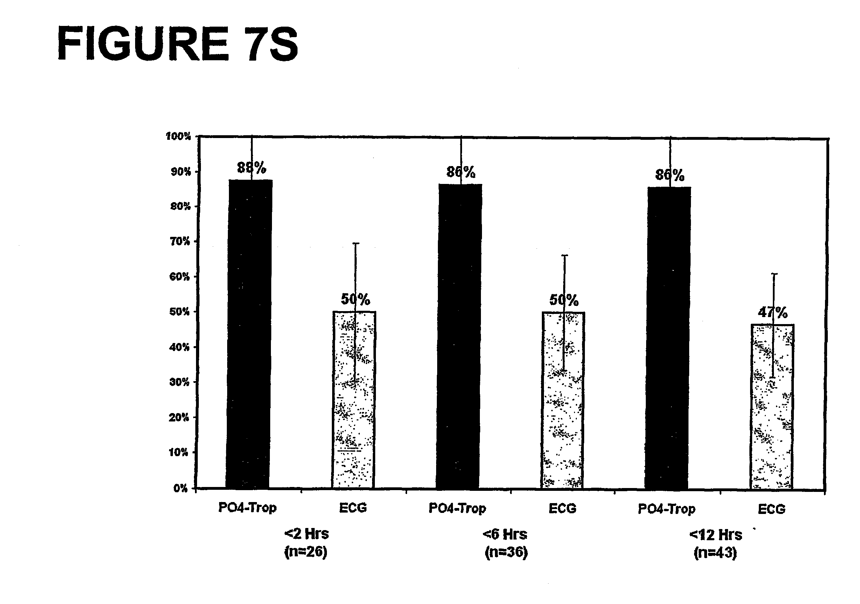

Figure 7A shows the low sensitivity and high specificity of the Dade Behring Dimension cTnI test for ACS. Figure 7B shows that the sensitivity of the cTnI test for ACS is very low in the first hours after the onset of ACS. Figure 7C shows the increased sensitivity achieved by combining the results of an ECG and the cTnI test for ruling out ACS. Figure 7D shows the increased sensitivity achieved by combining the results of an ECG and the cTnI test for ruling out ACS in the early hours after onset of ACS. Figure 7E shows a comparison of the sensitivity and specificity of the cTnI and PO₄-cTnI tests for all patients (n = 67). Figure 7F shows a comparison of the sensitivity of the cTnI and PO₄-cTnI tests in the first hours after onset of symptoms (the number of patients vary since the tests were performed only on the first blood drawn). Figure 7G shows a comparison of the specificity of the cTnI and PO₄-cTnI tests in the first hours after onset of symptoms. Figure 7H illustrates the increased sensitivity for ruling out ACS (all patients, n = 67) achieved by combining the ECG, cTnI and PO₄-cTnI results as compared to each test alone or the combination of ECG and cTnI. Figure 7I illustrates the increased sensitivity for ruling out ACS in the first hours after onset of symptoms (all patients, n = 67) achieved by combining the ECG, cTnI and PO₄-cTnI results as compared to a combination of ECG and cTnI results. Figure 7J shows the increased sensitivity for ACS achieved by combining the cTnI and PO₄-cTnI results for the STEMI subgroup of patients (n =17). Figure 7K shows the much greater sensitivity of the PO₄-cTnI test for ruling out ACS and the increased sensitivity for ruling out ACS achieved by combining the ECG, cTnI and PO₄-cTnI results for the UA and non-STEMI subgroup of patients (n = 21). Figure 7L shows the increased sensitivity for ruling out ACS achieved by combining the ECG, cTnI and PO₄-cTnI results as compared to the combination of ECG and cTnI for the UA and non-STEMI subgroup of patients (n = 21) in the early hours after onset of symptoms. Figure 7M shows the higher sensitivity of the PO₄-cTnI test for ruling out ACS and increased sensitivity for ruling out ACS achieved by combining the cTnI and PO₄-cTnI results for the low risk ECG subgroup of patients (n = 42). Figure 7N shows the 100% sensitivity of the PO₄-cTnI test for ruling out ACS and the lack of increased sensitivity for ruling out ACS achieved by combining the cTnI and PO₄-cTnI results for the UA subgroup of patients (n = 10). Figure 7O shows the specificity of ECG and PO₄-cTnI for ACS in the first hours after the onset of symptoms. Figure 7P and 7Q show the low sensitivity and high specificity of ECG for ACS. Figure 7R shows a comparison of the sensitivity and specificity of ECG and PO₄-cTnI for ACS for all patients (n = 67). Figure 7S shows the much higher sensitivity of the PO₄-cTnI test for ACS as compared to ECG for all patients (n = 67).

As can be seen from Figures 7A-7S, the measurement of PO₄-cTnI exhibits very high sensitivity as compared to cTnI, ECG and combinations of these two parameters, especially in the first 12-24 hours after the onset of symptoms. The specificity achieved with PO₄-cTnI was also very high, although not as high as that achieved by cTnI or ECG.

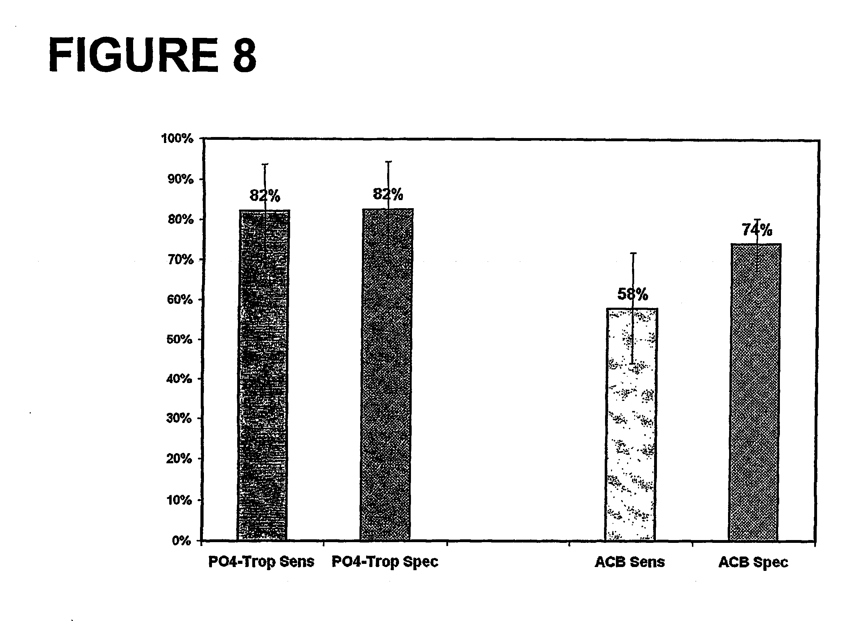

Figure 8 shows a comparison of the sensitivities and specificities of the PO₄-cTnI assay of the invention with the ACB™ test (Ischemia Technologies, Arvada, Colorado). The sensitivities and specificities for the ACB™ test are weighted (adjusted for the number of patients in each study) averages of those reported in Wu et al., Cardiovasc. Toxicol, 1:1-5 (2001) and Christenson et al., Clin. Chem., 47:464-470 (2001). The ACB^{™} test is a colorimetric assay approved by the Food And Drug Administration (FDA) for ruling out cardiac ischemia. Changes in the binding of cobalt to albumin are measured. As can be seen from Figure 8, the PO₄-cTnI assay of the invention exhibited both much higher sensitivity and much better specificity than seen with the ACB™ test.

### EXAMPLE 5: Diagnosis and monitoring of placental ischemia

Albumin, the most abundant plasma protein, exists as a collection of many species which are largely the result of post-translational modifications. It is possible to visualize these species accurately using high resolution mass spectroscopy and to quantify the relative amounts of each of the species yielding an albumin profile.

Plasma samples were collected from 17 pregnant women, 12 of whom had pregnancies affected by fetal growth restriction (FGR). Subjects for the study were selected from patients referred to a Maternal-Fetal Medicine (MFM) practice with complicated pregnancies. Inclusion criteria for the pilot study were:

Estimated fetal weight < 10^{th} percentile for gestational age by ultrasound in addition to:
- an amniotic fluid index (AFI) < 8 or,
- a ratio of blood flow velocity during systole to diastole (S/D) in the umbilical artery as measured with pulse-wave Doppler > 3 or,
- preeclampsia, as defined by standard clinical criteria.

There were 12 patients in the study group including 11 singletons and one twin gestation. There were 5 patients in the control group including 1 twin gestation. Gestational ages in the study group at time of delivery were between 26.3-38 weeks with an average gestational age of 30.2 weeks versus 38 weeks in the control group. Average birth weights were 1016 grams in the study versus 3114 grams in the control group. Birth weight percentages for the study group averaged < 10% versus 43% in the control group. Umbilical artery Doppler flow studies were obtained in 10 of the 12 study patients; of these, all were abnormal, with 2 patients having reversed end-diastolic flow, 6 having absent end-diastolic flow, and 2 having an S/D ratio > 3.0. Nine of the 12 study patients had preeclampsia. Two of the 12 study patients had HELLP syndrome.

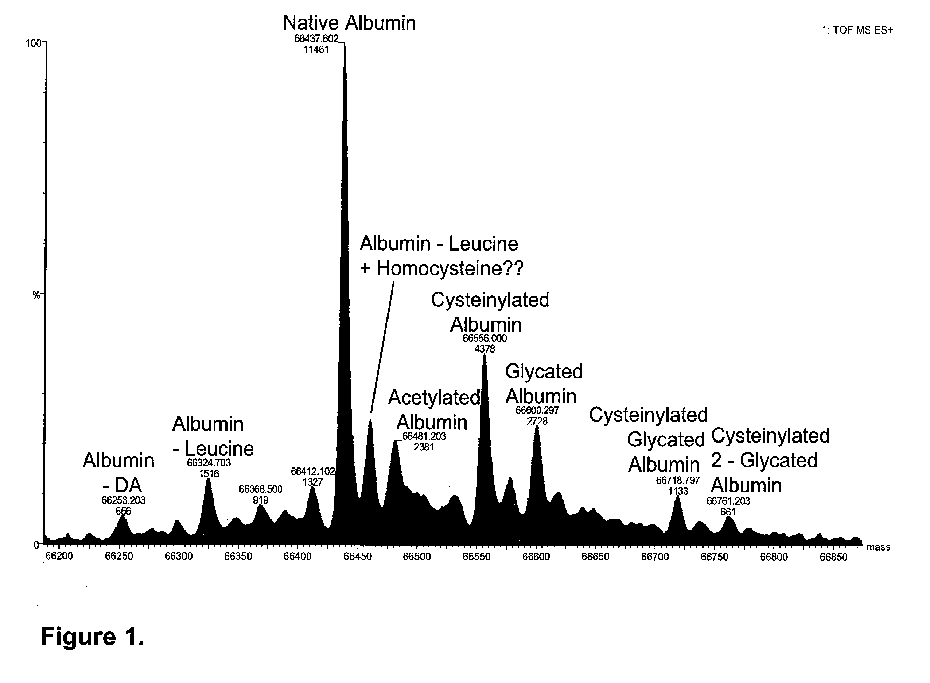

A sample albumin profile from a normal patient is shown in Figure 1. Note the many different species, each identified below:
- Native albumin
- Cysteinylated native albumin (inhibition of cystathionine beta synthetase)
- Native albumin with the C-terminal leucine missing (increased carboxypeptidase activity)
- Native albumin with the N-terminal aspartate-alanine missing (cyclization ofN-terminus)
- Cysteinylated native albumin with the C-terminal leucine missing
- Cysteinylated native albumin with the N-terminal aspartate-alanine missing
- Glycated native albumin - up to 3 glycations (hyperglycemia stress related)
- Cysteinylated, glycated native albumin
- Phosphorylated native albumin (increased surface kinase activity)
- Native albumin with the C-terminal leucine missing plus homocysteine.

Seven species of albuminwere identified that appearedto differ between women with placental ischemia and normal pregnancies. The percentage of total albumin each of these species represented was calculated. Below is a summary of the species found and their relative proportions (Table 5).

**TABLE 5: Relative levels of various species of albumin in a pilot study of placental ischemia**

| Patient ID | Diagnosis | Native Alb_{.}¹³ | +Cys¹⁴ | -Leu¹⁵ | -DA¹⁶ | Glycate d | Glycated, +Cys | -Leu, +Homocys ??¹⁷ |
|---|---|---|---|---|---|---|---|---|
| P006 | Normal | 49.80% | 22.49% | 2.55% | 2.71% | 8.86% | 3.99% | 9.59% |
| P007 | Normal | 46.22% | 24.57% | 3.49% | 2.26% | 8.97% | 4.73% | 9.75% |
| P011 | Normal | 41.74% | 33.06% | 2.08% | 1.52% | 8.62% | 4.48% | 8.50% |
| P013 | Normal | 49.73% | 21.14% | 3.32% | 2.62% | 9.46% | 4.18% | 9.54% |
| P018 | Normal | 57.61% | 13.23% | 5.36% | 2.87% | 8.49% | 2.19% | 10.25% |
| | | | | | | | | |
| P001 | PI¹⁸ | 15.21% | 61.43% | 1.25% | 1.02% | 8.26% | 8.44% | 4.39% |
| P002 | PI | 45.51% | 28.18% | 2.45% | 2.26% | 8.38% | 5.06% | 8.17% |
| P003 | PI | 43.06% | 32.73% | 2.54% | 1.58% | 7.79% | 4.89% | 7.41% |
| P004 | PI | 30.60% | 46.61% | 1.44% | 1.37% | 7.23% | 6.41% | 6.34% |
| P005 | PI | 8.86% | 70.42% | 0.97% | 0.63% | 7.04% | 8.68% | 3.40% |
| P008 | PI | 37.07% | 35.33% | 3.01% | 0.50% | 9.21% | 6.19% | 8.69% |
| P009 | PI | 38.70% | 34.51% | 2.73% | 1.84% | 8.26% | 5.57% | 8.39% |
| P012 | PI | 32.05% | 41.30% | 2.04% | 2.04% | 8.56% | 6.66% | 7.35% |
| P014 | PI | 33.88% | 40.37% | 1.89% | 1.62% | 8.74% | 6.50% | 6.99% |
| P015 | PI | 42.37% | 29.50% | 5.14% | 2.00% | 8.22% | 4.85% | 7.92% |
| P016 | PI | 27.70% | 47.28% | 1.92% | 1.57% | 7.82% | 6.88% | 6.84% |
| P017 | PI | 6.08% | 69.17% | 1.10% | 0.72% | 7.32% | 11.44% | 4.16% |

| | | | | | | | | |
|---|---|---|---|---|---|---|---|---|
| ¹³ Alb. = albumin ¹⁴ +Cys = cysteinylated ¹⁵ -Leu = missing the C-terminal leucine ¹⁶ -DA = missing the N-terminal two amino acids, aspartic acid and alanine ¹⁷ +Homocys = homocysteinylated ¹⁸ PI= placental ischemia | | | | | | | | |

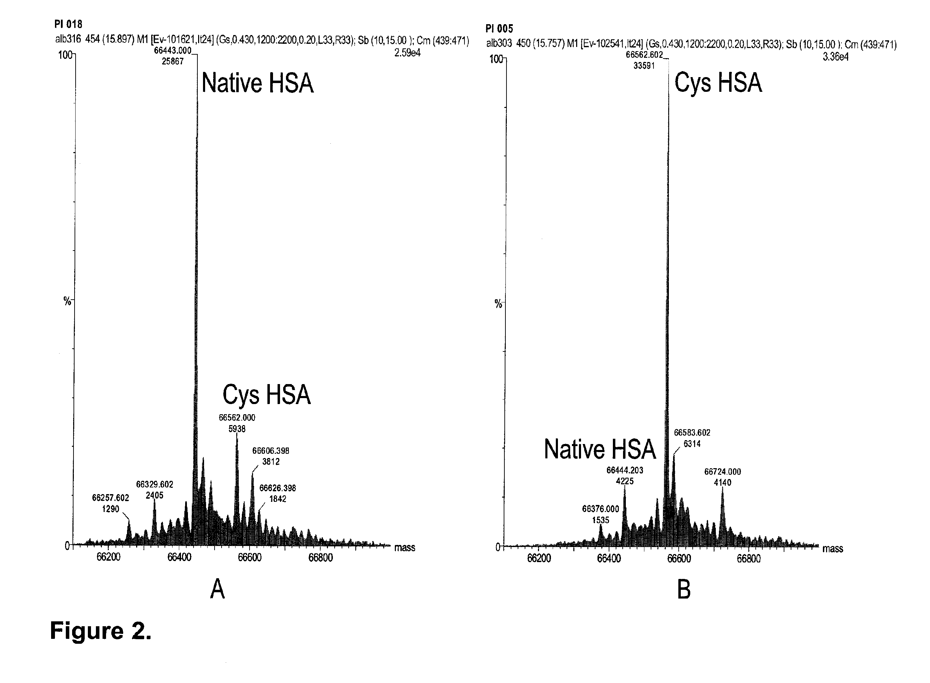

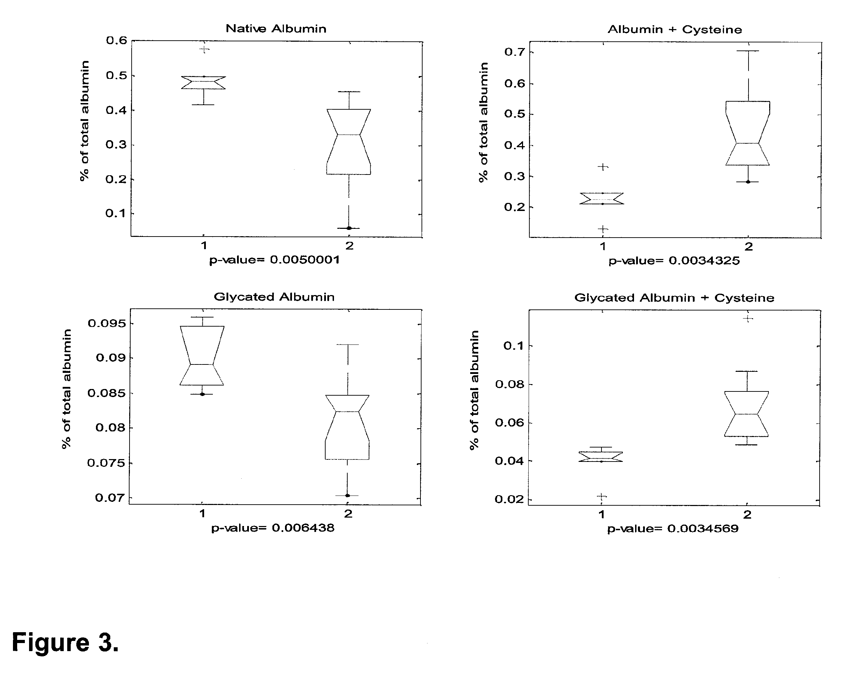

The two groups (normals and placental ischemia) were compared to see if there was a significant difference. A two-sided student's t-test was used. It was noted visually that the most apparent difference between the groups was the ratio of native to cysteinylated albumin. See Figure 2, which shows an example of the shift to cysteinylation from a normal pregnancy to a pregnancy with placental ischemia. Note the complete inversion of the native to the cysteinylated form. The difference in the level of cysteinylated albumin in normal pregnancies as compared to pregnancies with placental ischemia was found to be highly statistically significant (see Figure 3).

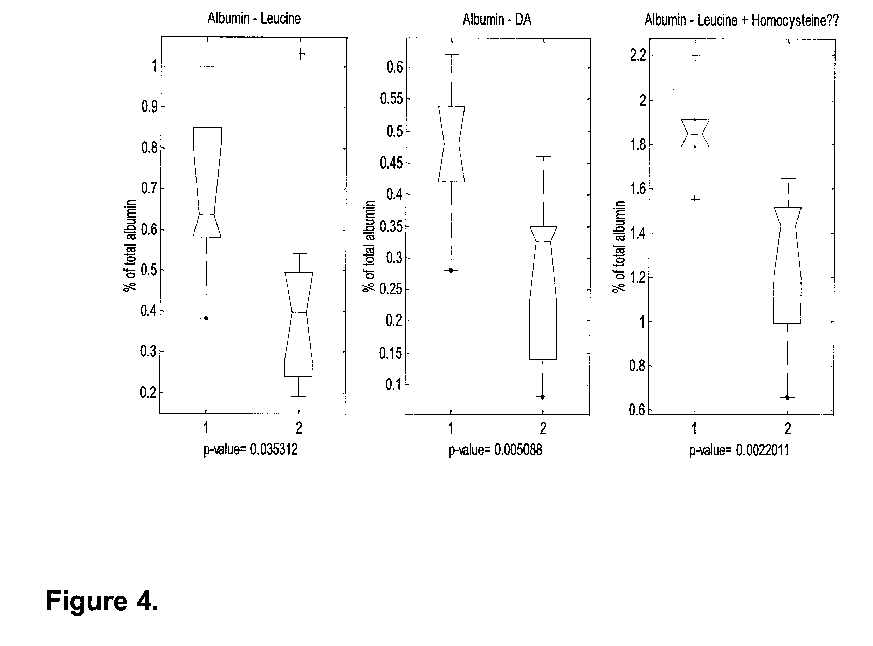

Since the most apparent difference between the groups was the ratio of native to cysteinylated albumin and, since relative ratios were being analyzed, it was reasoned that this large difference might influence the relative percentages of the others. To minimize this phenomenon, and to see if the differences in the remaining markers were truly significant, the data were renormalized by removing the cysteinylated albumins and their corresponding native species (see Figure 4).

Figures 3 and 4 present a set of boxplots that show the difference between the two groups (normal versus placental ischemia) as well as the p-values. Note that all of the 7 initial markers appear to be significant.

The highly significant elevation of cysteinylated albumin in placental ischemia can be explained as follows. The known elevation of plasma copper, homocysteine and cysteinylated albumin during peripheral vascular disease and ischemia can be unified through the transulphuration pathway (Figure 5). The key enzyme in the regulation of this pathway is cystathionine β-synthetase (CBS). This enzyme is deficient in homocysteinurea and is a heme-containing enzyme which maybe a redox sensor. Banerjee, R., et al., Biochim Biophys Acta, 2003. 1647(1-2): p. 30-5. Evidently, under oxidizing conditions, the activity of this enzyme is enhanced. By contrast, under reducing conditions, activity is decreased. If the enzyme is inhibited during placental ischemia (reducing conditions), it would explain the elevation of plasma homocysteine which, in turn, would be converted, under the oxidizing conditions of the mother, to cysteine, giving rise to elevated levels of cysteinylated albumin in the maternal circulation. While a correlation between plasma total homocysteine and copper in patients with peripheral vascular disease has been reported (Mansoor, M.A., et al., Clin Chem, 2000. 46(3); p. 385-91) it is not yet known if this occurs after placental ischemia. If this is indeed the case, then it is possible that the known redox flux between Cu(I) and Cu(II) per se or even the generation of free radicals from copper and plasma ascorbate (Bar-Or, D., et al., Biochem Biophys Res Commun, 2001. 284(3): p. 856-62) may also influence the direction of flow through the transulphuration pathway. In this way the increased plasma free copper alone could lead to the sequential elevation of plasma homocysteine, followed by conversion to cysteine and the generation cysteinylated albumin. The transulphuration pathway would explain the elevated levels of cysteinylated albumin observed in patients with placental ischemia.

Other proteins that are placenta- or fetus-specific, such as β-human chorionic gonadotropin (βHCG), alpha-fetoprotein (AFP), and pregnancy-associated protein 1A (PAP1A), will be analyzed for cysteinylation and other post translational modifications, such as acylation, phosphorylation, glycosylation, and nitrosylation. The above mentioned proteins will be extracted from maternal plasma, amniotic fluid and venous fetal cord plasma and analyzed by LC ESI/MS for the presence of the various species of interest (acetylation, phosphorylation, glycation, cysteinylation, and nitrosylation). In particular, the cysteinylation, phosphorylation and nitrosylation pathways are associated with ischemia and inflammation, and the levels of cysteinylated, phosphorylated and/or nitrosylated proteins (e.g., albumin, βHCG, AFP and PAP1A) are expected to be indicative of the degree of ischemia.

### EXAMPLE 6: Diagnosis and monitoring of placental ischemia by ELISA

The elevation of maternal cysteinylated albumin levels can be detected and quantified by Enzyme-Linked Immunosorbant Assay (ELISA) using antiserum raised against a peptide containing the cysteinylated cysteine residue of human serum albumin. The antiserum will be made as follows. A peptide will be synthesized corresponding to amino acids 28 to 41 of human serum albumin. Heating the peptide in a slightly alkaline environment in the presence of free cysteine will lead to cysteinylation of residue 34, a cysteine. Verification of peptide cysteinylation will be performed by mass spectrometry and, then, the antigen will be conjugated to a carrier protein. A primary immunization of 250 µg of antigen will be injected into two rabbits followed by three additional injections of 100 µg. Day 70 serum from both animals will be examined for reactive antibodies.

The cysteinylated albumin ELISA will be developed using the rabbit antiserum from above and an anti-human serum albumin capture antibody. Maternal serum albumin from patient plasma and/or serum will be captured using anti-human serum albumin antibodies coated onto ELISA strip well plates. To quantify the cysteinylated albumin present in the wells, sequential incubations of the anti-cysteinylated albumin antiserum and anti-rabbit IgG conjugated to horseradish peroxidase will be performed. ELISA validation and detection limits will be determined using known levels of cysteinylated albumin generated *in vitro*.

Antibodies used for the ELISA will be properly matched to ensure that one does not mask the binding site of the other. Selection of capture antibodies with antigenic sites away from the cysteinylation site will increase the chance of cysteinylated albumin capture. In addition, cysteine residue 34 was chosen for antibodyproduction, but additional residues may be involved. Cysteine 34 of human serum albumin is the only free cysteine on the native protein, but other cysteine residues may be exposed during an ischemic event.

Also, treatment with folates may affect the levels of cysteinylated albumin. Folates are known to remethylate homocysteine into methionine, hence avoiding the formation of cysteine. The amounts of folates ingested will be taken into consideration.

Other proteins that are placenta- or fetus-specific, such as βHCG, AFP, PAP1A, will be analyzed by ELISA for cysteinylation and other post translational modifications, such as phosphorylation and nitrosylation. Cysteinylation, phosphorylation and nitrosylation pathways are associated with ischemia and inflammation, and the levels of cysteinylated, phosphorylated and/or nitrosylatedproteins (*e.g.*, albumin, βHCG, AFP and PAP1A) should be indicative of the degree of ischemia.

### EXAMPLE 7: Monitoring of pregnancies for early detection of placental ischemia

It is expected that elevated levels of some ischemia biomarkers will antedate clinically apparent FGR. Therefore, patients with various risk factors and women with normal pregnancies will be selected for serial sampling during the pregnancy.

The following inclusion criteria will be used to identify pregnancies at risk for FGR:
- Previous pregnancy with severe intrauterine growth retardation (IUGR) neonate (< 5^{th} percentile);
- Previous intrauterine fetal distress (IUFD) with fetal weight < 10^{th} percentile;
- Moderate or high levels of anticardiolipin IgG;
- Renal disease with or without hypertension with serum creatinine (SCr) > 1.5 mg/dl; and
- Nulliparity and maternal side alpha-fetoprotein (MSaFP) and/or maternal side human chorionic gonadotropin (MShCG) >= 3.0 multiples of the median (MOM) on second trimester screen.

Normal control pregnancies will be selected from amongst women presenting for routine dating ultrasounds without any of the following clinical characteristics:

Estimated fetal weight < 10th percentile for gestational age by ultrasound in addition to:
- An amniotic fluid index (AFI) < 8 or
- A ratio of blood flow velocity during systole to diastole (S/D) in the umbilical artery as measured with pulse-wave Doppler > 3 or
- Preeclampsia, as defined by standard clinical criteria and without other risk factors for placental insufficiency, including:
- Previous pregnancy with severe IUGR neonate (< 5th percentile);
- Previous IUFD with fetal weight < 10th percentile;
- Moderate or high levels of anticardiolipin IgG;
- Renal disease with or without hypertension with SCr > 1.5 mg/dl;
- MSaFP and/or MShCG>=3.0 MOM on second trimester screen; and
- Identified prothrombotic condition (deficiency of protein S, protein C or antithrombin III or common mutations of Factor V, Factor II or methyltetrahydrofolate reductase (MTHFR)).
Control pregnancies delivering FGR neonates will be excluded post hoc.

Using these criteria, three populations will emerge: Study patients delivering FGR infants, study patients delivering AGA infants, and control patients delivering AGA infants. These three populations will be analyzed separately.

Study (at-risk pregnancies) and control patients will be identified at 16-20 weeks gestation. Samples of maternal serum (at 16-20, 24-28 and 32-36 weeks gestation) and of newborn venous and arterial cordblood will be collected in a gestational age matched fashion +/- 2 weeks. When study or control patients undergo amniocentesis for clinical indications, an aliquot of 1-2 cc of amniotic fluid will also be collected for analysis.

Samples will be analyzed by mass spectrometry and ELISA for post-translationally modified proteins, including at least cysteinylated, phosphorylated and nitrosylated proteins.

### EXAMPLE 8: Cysteinylated Albumin Is A Marker Of Bowel Ischemia And An Early Marker Of Multiple Organ Failure

Homocysteine is a intermediate in the metabolism of methionine and is the precursor of cysteine in the transulfuration pathway (Figure 5). Cystathionine β-synthetase (CBS) is the key regulatory enzyme in the transulfuration pathway (Figure 5). CBS may be a redox sensor. Evidently, its activity is decreased in a reducing environment (↓pH, low O₂) and increased in an oxidizing environment (↑pH, ↑O₂). Its activity should, therefore, be inhibited during ischemic events, which are characterized by acidosis and low oxygen (*i.e*., reducing conditions), and inhibition of CBS should cause the accumulation of homocysteine in ischemic tissue and hyperhomocysteinemia in the bloodstream. This hypothesis was tested in a rat model of bowel ischemia and multiple organ failure (MOF).

Ten control pathogen-free male Sprague Dawley CD rats were anaesthetized with isoflurene followed by an intravenous injection of sodium pentoparbitone (60 mg/kg body weight). The trachea of each rat was cannulated with polyethylene tubing, and the right jugular vein was cannulated with polyethylene tubing for administration of additional pentobarbitone as required. A midline abdominal incision was made and resutured Two hours post surgery, the sutures were removed and closed back by clamping with Spencer Wells. The rats were kept anesthetized for an additional hour, after which time they were exsanguinated and sacrificed.

Ten experimental pathogen-free male Sprague Dawley CD rats underwent the same procedure as the control group, except that a small metal artery clip was placed onto the superior mesenteric artery prior to suturing the abdominal incision. The metal clip was removed after 2 hours, and the abdominal incision closed back with Spencer Wells. The experimental rats were exsanguinated 1 hour post removal of the artery clip and sacrificed.

The blood was collected in heparinized tubes and spun at 4000 rpm for 10 minutes at 4°C to generate plasma. The plasma samples were frozen at -80°C for later analysis.

Homocysteine levels were determined using the IMMULITE 2000 Analyzer and kit for the quantitative determination of L-homocysteine.

Total albumin was measured using the bromocresol green (BCG) assay (Sigma Diagnostics, St. Louis, MO). Briefly, 5 µl of plasma were mixed with 1 ml of albumin reagent (BCG). Measurement was at 628 nm versus albumin reagent blank in duplicate using a Shimadzu UV160U spectrophotometer.

Albumin was isolated using the SwellGel® Blue Albumin Removal Kit (Pierce, Rockford, IL). Briefly, 100 µl of plasma were added to the column containing hydrated Cibacron® Blue resin. The column was rinsed with binding/wash buffer, and albumin was eluted with 1 M NaCl. The samples were desalted on Microcon 30 filters (Millipore, Bedford, MA) by rinsing with distilled water.

ESI and LC MS for albumin analysis were performed using a Waters 2794 HPLC system using a C-4 250 mm analytical column and a gradient system of water/trifluoroacetic acid (TFA) acetonitrile/TFA and a Micromass LCT mass spectrometer in +ESI. The percentage cysteinylation was calculated from total albumin species area under the curve in the resulting mass spectrogram.

The experimental animals had a 2.5 fold greater level of homocysteine in theirplasma than the control animals (p=0.00000017). The levels were 8.5 ± 1.5 µM for experimental animals and 3.3 ± 1.1 µM for control animals.

There was no difference in the albumin levels of the two groups. The albumin levels were 24.3 ± 2 and 23.1 ± 1.9 mg/ml for the control and experimental groups, respectively.

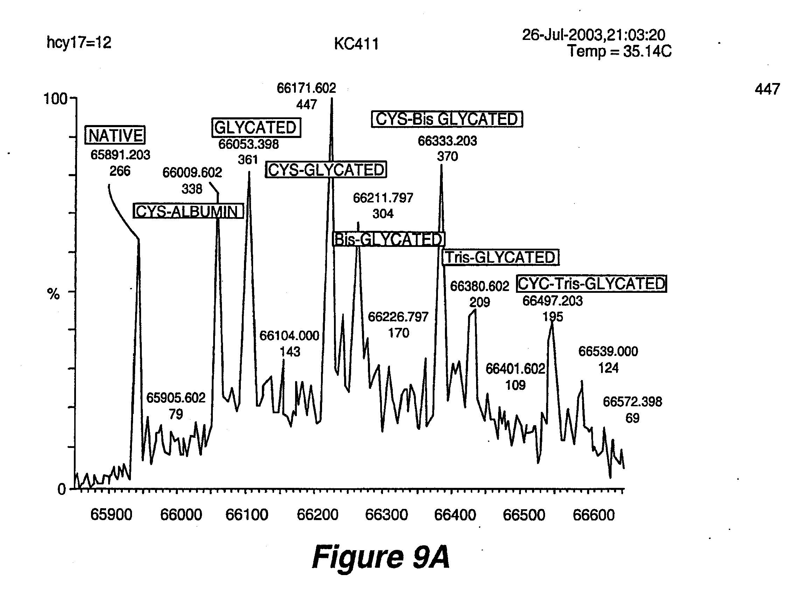

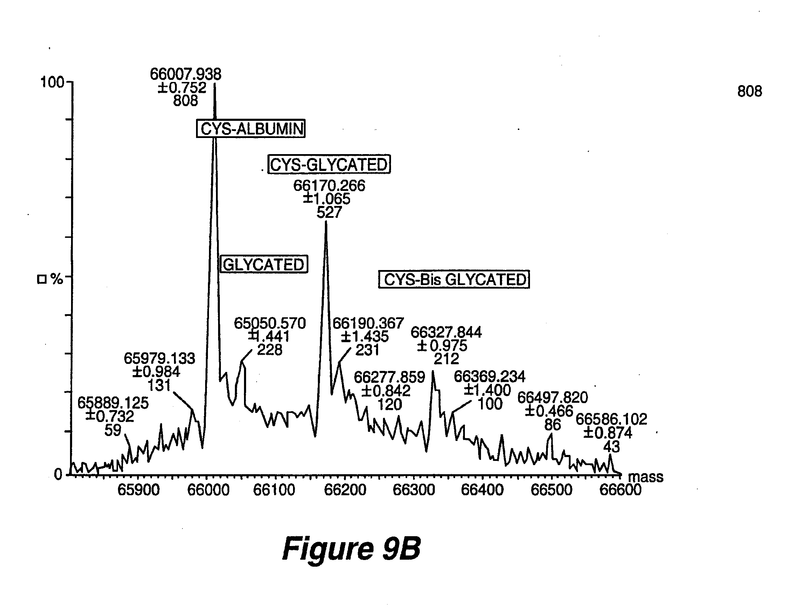

As shown by LC-MS and ESI/MS, all of the albumin of the experimental animals was present as various cysteinylated species (*i.e.*, 100% of the albumin was cysteinylated). In the control group, only 51.15 ± 8.77% of the total albumin was cysteinylated. Typical MS profiles of albumin from a control rat and an experimental rat are presented in Figures 9A and 9B. Superior mesenteric artery ligation results in severe bowel ischemia. This ligation alone, and this ligation in combination with reperfusion of the ischemic gut, have been used both as models of gut ischemia and for the induction of MOF.

In this study, it was demonstrated that gut ischemia and reperfusion are associated with a significant increase in plasma homocysteine levels. This homocysteine is presumably formed in the ischemic gut and subsequently liberated into the general circulation. Homocysteine in the presence ofoxidants in the normal peripheral circulation is metabolized into cysteine which, in turn, is transformed into cystine (Figure 5). Cystine, in the presence of free thiols, cysteinylates proteins.

The formation of cysteinylated species of albumin has been shown conclusively in the experimental group. The physiological significance of cysteinylation of the albumin cannot be underestimated. For instance, the free thiol of Cys34 of human albumin accounts for more than 90% of the plasma antioxidant reducing capability. Oxidizing these free thiol groups by cysteinylation severely reduces the buffering ability of plasma to handle oxidative stress and contributes to tissue damage.

The data show that cysteinylated albumin is a biomarker of bowel ischemia. Further, the quantity of the cysteinylated albumin may serve as an indicator of the severity of the ischemic insult (*i.e*., the greater the amount of cysteinylated albumin, the more severe the ischemia).

The high levels of the intermediate homocysteine together with the release of copper from carrier proteins during ischemic events contribute to the tissue damage observed. In the presence of copper, which is prevalent in acidotic/ischemic tissue, homocysteine-copper complexes, which are harmful to endothelial cells and potentially other cell lines, will be formed.

As noted above, the superior mesenteric artery ligation model is an accepted model of multiple organ failure (MOF); the rats will develop MOF after the reperfusion phase. Thus, cysteinylated albumin also appears to be an early marker for MOF.

This conclusion is supported by the fact that homocysteine levels in human MOF patients have been measured and have been found to be elevated 1,5-fold to 2.0-fold. For instance, one patient had a homocysteine level of 16.7 µM while suffering from MOF. The homocysteine level dropped to 3.1 µM after intensive treatment and recovery of the patient from MOF (homocysteine levels for normal humans are 5-12 µM).

### EXAMPLE 9: Improved Method Of Diagnosing Appendicitis

Abdominal pain is one of the most frequent complaints of patients in the emergency department and physicians' offices, and appendicitis is the most common cause of acute abdominal pain requiring emergency surgical intervention. Graff et al., Acad. Emerg. Med., 7:1244-1255 (2000); Berry et al., Ann. Surg., 200:567-575 (1984). Emergency physicians and family physicians, rather than surgeons, usually perform the initial clinical evaluation of patients with suspected acute appendicitis. The correct diagnosis of acute appendicitis relies heavily on the skill and experience of the clinician. Sternbach et al., J. Emerg. Med., 13:95-96 (1995); Hale et al., Ann. Surg., 225:252-261 (1997). Subtle and atypical signs and symptoms often lead to misdiagnosis, and diagnostic delays can lead to adverse outcomes. Jahn et al., Eur. J. Surg., 163:433-443 (1997); Andersson et al., Eur. J. Surg., 166:796-802 (2000). A recent multi-center review reported an initial misdiagnosis rate of approximately 20% for appendicitis in emergency department patients with abdominal pain and without extended observation. Graff et al., Acad. Emerg. Med., 7:1244-1255 (2000). Women and children have noticeably higher initial misdiagnosis rates for acute appendicitis. Rothrock et al., J. Emerg. Med., 13:1-8 (1995); Rothrock et al., Ann. Emerg. Med., 36:39-51 (2000).

Orthohydroxyhippuric acid (OHHA) is a normal constituent of human urine. Preliminary clinical observations suggested a possible association between increased urinary OHHA and acute appendicitis. In this prospective, non-interventional study of emergency department patients with acute abdominal pain, the diagnostic a value of a rapid, simple test for urinary OHHA (AppyTest® assay, DMI BioSciences, Inc., Englewood, Colorado) alone and in combination with the initial leukocyte count in the early clinical assessment of suspected acute appendicitis was investigated. In a cost-conscious healthcare environment, simple diagnostic tests that increase specificity and diagnostic accuracy can assist clinicians to efficiently and economically assess patients with suspected acute appendicitis.

### A. Methods And Materials

Consecutive emergency department patients presenting with acute abdominal pain suspicious for appendicitis were enrolled 24 hours per day in this prospective study at three urban and suburban community referral hospitals with teaching affiliations: HealthOne Swedish Medical Center, Englewood, CO, Exempla St. Joseph's Hospital, Denver, CO, and Centura Health Porter Adventist Hospital, Denver, CO. Institutional review board approval was obtained at each center, and informed consents were obtained from all patients or their authorized representatives. Inclusion criteria were as follows: patients of any age, symptoms that included recent-onset abdominal pain, and an initial history and physical examination determined suspicious for acute appendicitis by an experienced emergency physician specialist. Independent clinical study representatives were responsible for patient interviews, specimen handling, and all study related data collection. Patient information, including historical and physical examination data, was recorded into a formalized data instrument during the initial emergency department examination and before any diagnostic test results were known.

Exclusion criteria were as follows: previous appendectomy, diagnosed pre-morbid abdominal pathology (*e.g*. trauma, pancreatitis, bowel obstruction, threatened abortion, gastrointestinal bleeding), clinical jaundice, metastatic cancer, history of abdominal surgery or complaints of abdominal pain within the preceding three months, or the onset of current symptoms > 72 hours prior to the examination. Phenolic compounds (*e.g*. phenothiazines) and salicylates can produce false positive OHHA results (Altschule et al., Clin. Pharmacol. Ther., 15:111-117 (1974)), and patients with a history of ingesting those compounds in the preceding 24 hours were excluded. Patients taking oral antibiotics in the preceding 24 hours were excluded because antibiotics could affect intestinal bacteria. Patients who had surgical interventions delayed more than 12 hours after the urine specimen collection were excluded from the study. Experienced and fully trained emergency and surgical physician specialists, blinded to OHHA assay results, made all diagnostic and treatment decisions according to local standards of care. To record clinical outcomes, study representatives conducted telephone interviews with patients within 7 days of the initial examination and reviewed each medical record.

All patient urine specimens were collected during the initial emergency department presentation and refrigerated at 4°C. Urine specimens were collected within 1 hour of the time the leukocyte count sample was obtained. A color assay for OHHA (AppyTest^{®} assay kit, DMI BioSciences, Inc., Englewood, CO) was completed in a blinded fashion within 1 to 7 days by a trained laboratory technician at an independent clinical laboratory (University of Colorado Health Sciences Center, Denver, CO). Briefly, the OHHA assaymethod consists of adding 15 drops of urine to a color reagent and mixing for 30 seconds; a color change from pink to dark brown or purple color (compared to a color chart) indicates a positive test. An OHHA assay visual detection threshold, with >95% reproducibility, was correlated by high performance liquid chromatography to 40 mg/L of OHHA in urine and the assay has acceptable stability, validity and reproducibility for up to 7 days in urine specimens refrigerated at 4°C (data not shown). Inconclusive urinary OHHA results were defined as non-standard color changes not found on the OHHA assay color chart and those patients were removed from the study. Physicians, medical staff, patients, clinical study representatives, and sponsors were all unaware of OHHA assay results during the study. An independent study coordinator compiled all diagnostic and outcome study data from case report forms after the patients were enrolled, diagnosed and treated.

Automated total leukocyte counts were performed in each hospital's clinical laboratory during the emergency department presentation. Leukocyte count results were considered elevated at the following age-adjusted thresholds: ≥ 18 years, 12.0 x 10⁹/L; 11-17 years, 13.0 x 10⁹/L; 7-10 years, 13.5 x 10⁹/L; 4-6 years, 14.5 x 10⁹/L; 2-3 years, 15.5 x 10⁹/L (there were no patients younger than 2 years old). For the purposes of this study, age-adjusted leukocyte counts that were not elevated were considered "normal." Patients who did not have a recorded leukocyte count were excluded from the analysis.

Diagnostic indices (specificity, sensitivity, accuracy, and predictive values) for combinations of OHHA assay results and leukocyte counts were examined using the logical operator "AND" in comparison to diagnostic indices for the leukocyte count alone. Test results were considered concordant if a patient had either 1) "positively concordant" results - a positive OHHA result and with an elevated leukocyte count or 2) "negatively concordant" results - a negative OHHA result and a leukocyte count that was not elevated.

The diagnosis of acute appendicitis was confirmed by histopathological report for those patients having surgical intervention following the initial emergency evaluation. The presence of periappendicitis, inflammation of tissue around the appendix but no inflammation within the wall of the appendix, was considered negative for acute appendicitis. Pathologists performing the histopathological examination were unaware of OHHA assay results. All enrolled patients who were discharged without surgical intervention were contacted 1 to 7 days later and considered negative for acute appendicitis if they had no symptoms of acute appendicitis and had no exploratory abdominal surgical interventions within 12 hours following the initial examination.

Standard calculations for the diagnostic indices and 95% confidence intervals (CI) were performed with a computer program (Excel 2000®, Microsoft Corp and S-PLUS^{®}, Insightful Corp). OHHA assay and leukocyte count results were compared using the chi-square test of independence. Logistic regression was used to identify any additional value of the OHHA assay in relation to useful dichotomous variables. Statistical significance was established at the 0.05 confidence level.

### B. Results

Of 300 patients who were eligible and gave consent for participation during the seven-month study, 36 patients were excluded from analysis for the following reasons: 18 had inconclusive OHHA assay results, eight patients had no leukocyte count obtained during the initial clinical evaluation, and 10 patients had other missing data. Additionally, 55 patients who had surgical interventions delayed more than 12 hours after the urine OHHA assay were excluded from analysis leaving a study group of 209 patients. Of the 209 study group patients, 59 (28.2%) went directly from the emergency department for surgical intervention and 150 (71.8%) were discharged home. On follow-up, none of the patients discharged home had symptoms consistent with acute appendicitis or surgical explorations following the initial emergency department presentation. Demographic and diagnostic data are presented in Table 6. Of the 18 patients removed from the study for inconclusive OHHA assay results, three (16.7%) had appendicitis. Thirty-five of the 55 patients (63.6%) with delayed surgical interventions had appendicitis.

Diagnostic results of the patients who had immediate surgical intervention were as follows: 44/59 (74.6%) had acute appendicitis confirmed by histopathology, 8/59 (13.6%) had anormal appendix, and 7/59 (11.9%) had other surgical diagnoses that included subacute periappendiceal inflammation, inflammatory lymphoid hyperplasia, adhesions of the appendix, umbilical hernia, ovarian cancer, Meckel's diverticulitis, and paraovarian cyst. Seven of the 44 patients (15.9%) with acute appendicitis had perforated appendicitis confirmed by histopathology (Table 6).

The average age of the 44 patients with acute appendicitis was 28.3 years old (range 7 to 65); 33/44 (75.0%) were male, and 11/44 (25.0%) were female (Table 6). The prevalence of appendicitis for males was 33/82 (40.2%),11/127 (8.7%) for females, 31/158 (19.6%) for adults >15 years old and 13/51 (25.5%) for children ≤15 years old. In patients with acute appendicitis, the physician recorded that 39/44 (88.6%) had abdominal guarding, 27/44 (61.4%) had rebound abdominal tenderness, and 41/44 (93.2%) had abdominal pain located only in the right lower quadrant.

**TABLE 6**

| **PATIENT DEMOGRAPHICS** | | |
|---|---|---|
| | Male | 82/209 (39.2%) |
| | Female | 127/209 (60.8%) |
| | Average Age | 26.3 (Range: 4-79) |
| | Children (≤ 15 years) | 51/209 (24.4%) |

| **CLINICAL SIGNS AND SYMPTOMS** | | |
|---|---|---|
| | Nausea | 148/209 (70.8%) |
| | Guarding | 116/209 (55.5%) |
| | Anorexia | 112/209 (53.6%) |
| | Fever during the preceding 48 hours | 100/209 (47.8%) |
| | Vomiting | 80/209 (38.3%) |
| | Rebound tenderness | 71/209 (34.0%) |

| **PATIENTS HAVING SURGICAL EXPLORATION** | | |
|---|---|---|
| | Acute appendicitis* | 44/59 (74.6%) |
| | Other diagnoses (including normal appendix)* | 15/59 (25.4%) |
| | Normal appendix (only)* | 8/59 (13.6%) |

| **PATIENTS WITH APPENDICITIS** | | |
|---|---|---|
| | Perforated appendicitis* | 7/44 (15.9%) |
| | Male | 33/44 (75.0%) |
| | Female | 11/44 (25.0%) |
| | Children (≤ 15 years) | 13/44 (29.5%) |

| | | |
|---|---|---|
| * Diagnosis by histopathology | | |

Overall, the OHHA assay was positive in 53/209 (25.4%) patients and negative in 156/209 (74.6%) patients. The leukocyte count was elevated in 81/209 (38.8%) patients and normal in 128/209 (61.2%). Diagnostic indices of the OHHA assay and the leukocyte count are shown in Table 7. The OHHA assay specificity of 80.6% (95% CI, 73.6%-86.2%; p<0.01) was significantly higher than the leukocyte count specificity of 67.9% (95% CI, 60.1%-74.8%). The OHHA sensitivity of 47.7% (95% CI, 32.7%-63.1%) was not significantly different than the leukocyte count sensitivity of 63.6% (95% CI, 47.7%-77.2%). Of the seven patients whose abdominal surgery revealed another surgical diagnosis and a normal appendix, six (85.7%) had negative OHHA assay results. Urine specific gravity was recorded in 198 (94.7%) of the patients. Of the 26 patients with a urine specific gravity <1.010, 23 were true negatives, two were false negatives, and one was a true positive for acute appendicitis.

Analysis of the combination of both a positive OHHA assay "AND" an elevated leukocyte count as a positive result demonstrated significantly higher specificity of 92.1% (95% CI, 86.6%-95.6%; *p*<.001) than the specificity of 67.9% (95% CI, 60.1%-74.8%) for leukocyte count alone (Table 7). The same combination of test results also produced significantly higher diagnostic accuracy of 78.9% (95% CI, 72.7%-84.1%; *p*=0.006) compared to the accuracy of 67.0% (95% CI, 60.1%-73.2%) for the leukocyte count alone (Table 7). Sensitivity for the combination of both a positive OHHA assay "AND" an elevated leukocyte count was 29.5% (95% CI, 17.2%-45.4%; *p*=0.001), which was significantly lower than the sensitivity of 63.6% (95% CI, 47.7%-77.2%) for the leukocyte count alone (Table 7). Diagnostic indices for the OHHA assay and the same test combination of both a positive OHHA "AND" an elevated leukocyte count were determined for the following subgroups: males, females, patients > 15 years old, and patients ≤ 15 years old. For each subgroup, specificity and accuracy were not significantly different from the overall results.

Combinations of results of the two tests were positively or negatively concordant in 127/209 (60.8%) patients and not concordant in 82/209 (39.2%) patients. Concordant OHHA and leukocyte count results demonstrated significantly higher specificity of 87.7% (95% CI, 79.6%-93.1%; *p*<0.001) than the specificity of 67.9% (95% CI, 60.1%-74.8%) for leukocyte count alone (Table 8). Concordant OHHA and leukocyte count results also demonstrated significantly higher diagnostic accuracy of 83.5% (95% CI, 75.6%-89.2%; *p*<0.001) compared to the accuracy of 67.0% (95% CI, 60.1%-73.2%) for the leukocyte count alone (Table 8). Negatively concordant results, the combination of a negative OHHA assay and a normal leukocyte count, resulted in a negative predictive value of 92.1% (95% CI, 84.5%-96.3%); sensitivity and predictive values were not significantly different from the leukocyte count alone (Table 8).

Analysis of a subset of 96 patients provided a preliminary indication that a combination of an elevated OHHA and>10% immature "band" neutrophil leukocytes would also give improved diagnostic accuracy for appendicitis.

The goal was to include meaningful variables, solicited from each patient, in the regression equation and to exclude those variables that have little or no effect on the correlation with appendicitis. Chi-square tests of independence between the variable of acute appendicitis and the other dichotomous variables, if significant, may suggest variables that are not independent of the acute appendicitis outcome. Conversely, chi-square tests of independence between the variable OHHA and the other dichotomous variables, if not significant, may suggest variables that are independent of the OHHA outcome and thus will offer OHHA more of a chance to contribute to the prediction. If two variables are highly associated with each other (*e.g.* anorexia and nausea, or nausea and vomiting), then only one was included in the model. In this study, the variables anorexia, fever, nausea, vomiting did not appear to have any value in predicting acute appendicitis. Comparable to previous reports, the present study identified leukocyte count, gender, guarding, rebound, and abdominal pain location to be the most useful variables for prediction of a correct diagnosis (Andersson et al., World J. Surg., 23:133-140 (1999)). Adding the OHHA assay result to the useful variables in this study significantly reduced the residual log likelihood (deviance) score (114.51 vs.120.75, *p*=0.013), which indicated that the OHHA result contributed to the correct diagnosis when all of the useful variables are considered.

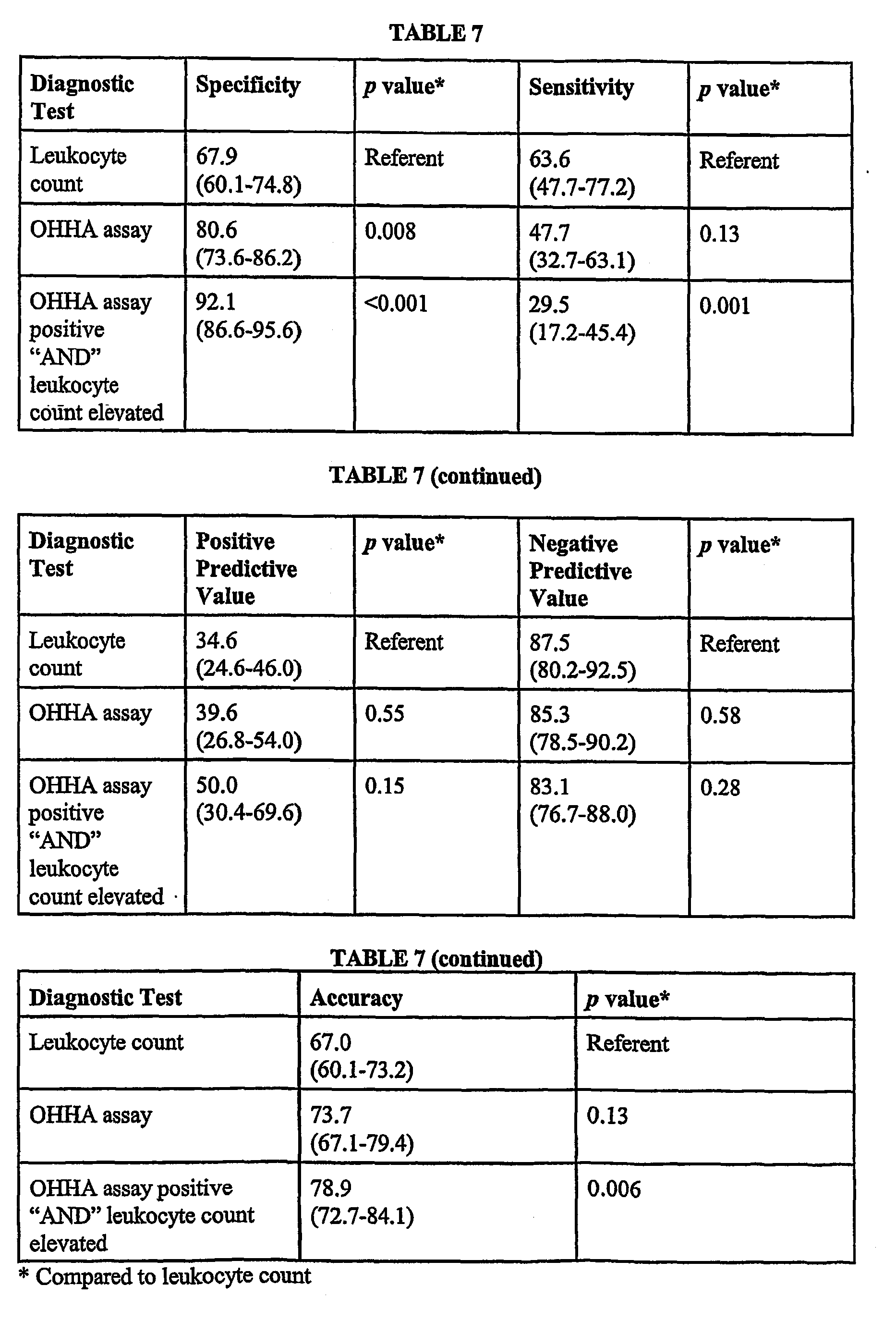

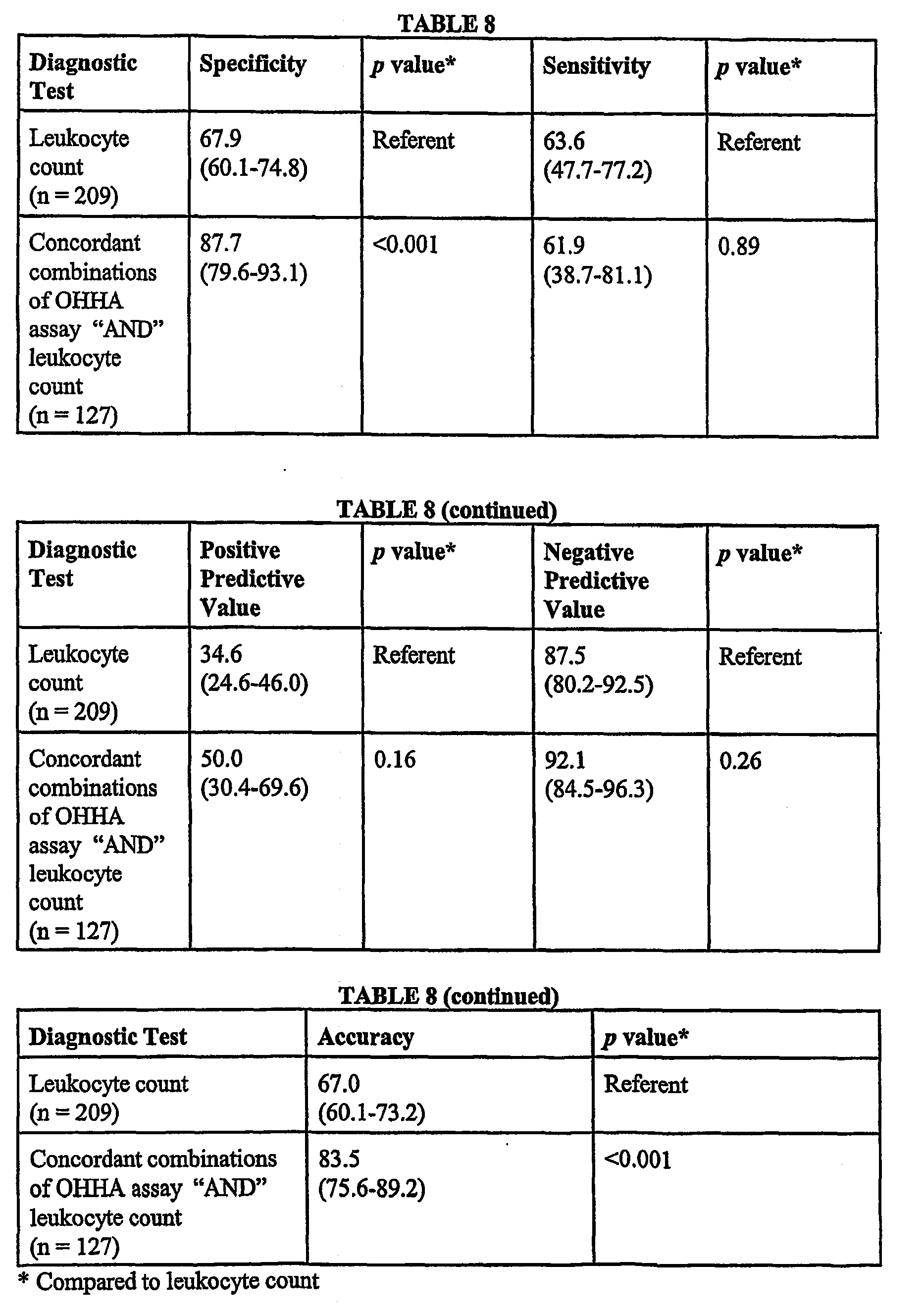

### C. Discussion

Emergency and primary care physicians examine numerous patients with abdominal pain, yet very few of those patients actually have appendicitis. Graff et al., Acad. Emerg. Med., 7:1244-1255 (2000). The accuracy of the initial evaluation of patients with suspected appendicitis depends on the skills, careful observation, and personal experience of the clinician. Sternback et al., J. Emerg. Med., 13:95-96 (1995); Andersson et al., World J. Surg., 23:133-140 (1999); Bolton et al., Br. J Surg., 62:906-908 (1975); Vermeulen et al., Eur. J. Surg.,161:483-486 (1995*).* No specific biochemical marker is currently available to confirm the diagnosis of acute appendicitis. Early clinical assessments depend on the integration of a history of pain, findings of abdominal tenderness, and the results of non specific markers of inflammation. Andersson et al., Eur. J. Surg., 166:796-802 (2000). Delays in the diagnosis of appendicitis can lead to increased morbidity and mortality. Consequently, clinicians performing the initial examination often order expensive radiological imaging tests, request emergency surgical consultations, or arrange for extended hospital observations. Stroman et al., Am. J. Surg., 178:485-489 (1999); Bachoo et al., Pediatr. Surg. Int., 17:125-128 (2001). Helical and contrast CT, and perhaps ultrasonography, may have high enough specificity and sensitivity to help confirm the diagnosis of appendicitis prior to surgical intervention. Stroman et al., Am. J. Surg., 178:485-489 (1999); Pickuth et al., Eur. J. Surg., 166:315-319 (2000); Franke et al., World J. Surg., 23:141-146 (1999). However, radiological imaging tests are not recommended for the routine screening of patients with abdominal pain because they are expensive, resource intensive, and not readily available in many clinical settings. Jahn et al., Eur J. Surg., 163:433-443 (1997); Franke et al., World J. Surg., 23:141-146 (1999); Sarfati et al., Am. J. Surg., 166:660-664 (1993).

As far as is know, this study is the first to associate increased urinary OHHA levels with improved specificity and diagnostic accuracy for clinically suspected acute appendicitis. The results of the present study indicate that a simple, rapid assay for a threshold concentration of urinary OHHA has a significantly higher diagnostic specificity than the initial leukocyte count in suspected acute appendicitis. The higher specificity for the OHHA assay was demonstrated both independently and in combination with the initial leukocyte count.

Numerous investigations have recommended use of the total leukocyte count as a diagnostic aid in the early clinical assessment of patients suspected of having acute appendicitis. Andersson et al., Eur. J. Surg., 166:796-802 (2000); Andersson et al., World J. Surg., 23:133-140 (1999); Dueholm et al., Dis. Colon Rectum, 32:855-859 (1989); Hallan et al., Eur. J. Surg., 163:533-538 (1997); Gronroos et al., Clin. Chem., 40:1757-1760 (1994). One report even suggests that inflammatory variables, such as the leukocyte count, contain diagnostic information equally important to the clinical findings. Andersson et al., World J. Surg., 23:133-140 (1999). However, despite broad acceptance, misdiagnosis of appendicitis due to both false negative and false positive leukocyte counts has been widely reported. Bolton et al., Br. J. Surg., 62:906-908 (1975); English et al., Am. Surg., 43:399-402 (1977); Coleman et al., Am. Surg., 64:983-985 (1998). The classic triad of a history compatible with appendicitis, pain at McBurney's point, and an elevated leukocyte count is reported to have a diagnostic accuracy of less than 80%. Gronroos et al., Clin. Chem., 40:1757-1760 (1994). Combining diagnostic test results to increase accuracy is a useful approach for diseases with equivocal signs and symptoms. Lin, J. Biopharm. Stat., 9(1):81-88 (1999); Kline et al., JAMA, 285:761-768 (2001). However, previous investigations combining the total leukocyte count with other laboratory parameters, such as C-reactive protein, phospholipase A2, neutrophil count, and band neutrophil count have not reported increased specificity for acute appendicitis. Goodman et al., Am. Surg., 61:257-259 (1995); Bolton et al., Br. J. Surg., 62:906-908 (1975); Dueholm et al., Dis. Colon Rectum, 32:855-859 (1989); Hallan et al., Eur. J. Surg., 163:533-538 (1997); Gronroos et al., Clin. Chem., 40:1757-1760 (1994); Oosterhuis et al., Eur. J. Surg., 159:115-119 (1993).

Results of the present study indicate that combining the OHHA. assay result (with significantly higher specificity) and the total leukocyte count (with higher sensitivity) improves the diagnostic accuracy of the initial clinical assessment of suspected acute appendicitis. The combination of a positive urinary OHHA result and an elevated initial leukocyte count resulted in significantly higher specificity and diagnostic accuracy than the leukocyte count alone. The combination of a negative OHHA assay result with a negative leukocyte count demonstrated a negative predictive value of 92%. From a clinical standpoint, it appears that concordant test results are the most useful test combinations to assist the evaluation of clinically suspected appendicitis. In this study, concordant test results occurred in a majority (60.8%) of the patients. OHHA assay and leukocyte count results that are not concordant indicate a need for further diagnostic testing to establish the presence or absence of appendicitis.

Overall prevalence rates for gender and age subgroups in this study were similar to other recent reviews of acute appendicitis. Hale et al., Ann. Surg., 225:252-261 (1997); Andersson et al., Eur. J. Surg., 166:796-802 (2000); Guss et al., Am. J. Emerg. Med., 18:372-375 (2000). Several reports have shown that women and pediatric patients with suspected appendicitis are more difficult to assess clinically. Rothrock et al., J. Emerg. Med., 13:1-8 (1995); Rothrock et al., Ann. Emerg. Surg., 36:39-51 (2000); Borgstein et al., Surg. Endosc., 11:923-927 (1997). Women of childbearing age have a noticeably higher misdiagnosis rate for appendicitis than men due to clinical presentations that mimic gynecological disorders. Andersson et al., Eur. J. Surg., 166:796-802 (2000); Rothrock et al., J. Emerg. Med., 13:1-8 (1995); Guss et al., Am. J. Emerg. Med., 18:372-375 (2000); Borgstein et al., Surg. Endosc., 11:923-927 (1997). Additionally, women have been shown to have longer delays in operative intervention, especially with perforated appendix. Rothrock et al., J. Emerg. Med., 13:1-8 (1995); Guss et al., Am. J. Emerg. Med., 18:372-375 (2000). Pediatric patients also have higher misdiagnosis rates, longer delays in diagnosis, and increased morbidity and mortality with acute appendicitis. Rothrock et al., Ann. Emerg. Surg., 36:39-51 (2000); Bachoo et al., Pediatr. Surg. Int., 17:125-128 (2001). In the present study, the combination of both a positive OHHA result and an elevated leukocyte count for each gender and age subgroup demonstrated increased diagnostic specificity and accuracy that were statistically comparable to the overall results. Improved specificity and accuracy with a combination of these tests could prove particularly useful for the early evaluation of women and pediatric patients with clinically suspected appendicitis.

Although results of the present study do not suggest that the OHHA assay alone is a confirmatory test for acute appendicitis, the OHHA assay can add significant incremental diagnostic value when combined with the initial leukocyte count. Contributory diagnostic tests provide diagnostic information that needs to be integrated into the clinical assessment. Andersson et al., World J. Surg., 23:133-140 (1999). Combinations of contributory tests, such as the OHHA assay and the initial leukocyte count, that significantly improve diagnostic accuracy can help reduce false negative and false positive clinical evaluations of suspected appendicitis. Rapid and relatively inexpensive tests that improve early diagnostic accuracy for appendicitis can also help optimize the cost-effective use of other diagnostic interventions such as radiological imaging tests, surgical consultations, extended hospital observations, repeat physical examinations, and serial laboratory tests. A high level of clinical acceptability for the OHHA assay is anticipated because it combines the convenient and noninvasive nature of urine sampling with a rapid assay method. Additionally, the simple OHHA assay method can be easily performed in any hospital or outpatient laboratory or at the bedside.

The present study has several limitations. Urine specimens were batched before performing the OHHA assay to facilitate standardized analysis by a limited number of laboratory technicians. Completing the assay within the first hour after collection would have more closely represented clinical utilization. OHHA assay results could have been affected by excessively dilute urine specimens; however, the present study was not designed to address that subset of results. Trends in the data suggest that a urine specific gravity ≥1.010 would be expected to provide more reliable results. The present study compared the OHHA assay and leukocyte count results to the coincident surgical diagnosis of acute appendicitis and excluded patients having delayed surgical intervention, many ofwhom were later diagnosed with appendicitis. Future studies of serial OHHA and leukocyte count results may help determine if the tests are useful for patients requiring prolonged observation. Spontaneous resolution of acute appendicitis in patients who did not have surgery has been previously described (Bolton et al., Br. J Surg., 62:906-908 (1975)) and could have affected results. The study was designed to evaluate the OHHA assay used in conjunction with the initial clinical assessment and did not include comparisons with confirmatory radiological procedures such as contrast or helical CT. Many patients in this study had total leukocytes counts ordered without a differential leukocyte count making any comparisons with neutrophil counts or band neutrophil counts impractical. Further studies are warranted to determine if the OHHA assay improves diagnostic value for acute appendicitis when combined with other markers of inflammation.

Results of this study indicate that an assay for the presence of increased urinary OHHA has significantly greater specificity than the total blood leukocyte count for patients with clinically suspected acute appendicitis. The combination of an elevated leukocyte count and a positive urinary OHHA assay resulted in significantly higher specificity and diagnostic accuracy than leukocyte count alone. Combining a negative OHHA result and a normal leukocyte count could assist the efficient and cost-effective use of healthcare resources in patients who have a lower probability of having appendicitis. The OHHA assay is a simple, non-invasive test that, combined with the leukocyte count or other marker of inflammation, can increase early diagnostic accuracy and aid the initial clinical assessment of suspected acute appendicitis.

### D. Summary

The initial evaluation of patients with suspected acute appendicitis remains a diagnostic challenge, and the total leukocyte count, despite having low specificity, is often used to assist clinical decisions. In 209 consecutive emergency department patients with suspected acute appendicitis, the diagnostic specificity of a novel assay for urinary orthohydroxyhippuric acid (OHHA) alone and in combination with the leukocyte count was prospectively investigated. Forty-four of 59 patients having immediate surgery had histopathologically confirmed acute appendicitis. OHHA assay specificitywas significantly higher than leukocyte count specificity (80.6% vs. 67.9%, *p*<0.01). Combining positive OHHA "AND" elevated leukocyte counts produced significantly higher specificity than leukocyte count alone (92.1% vs. 67.9%, *p*<0.0001). Concordant test results, a positive OHHA combined with an elevated leukocyte count or negative OHHA combined with a normal leukocyte count, had significantly higher specificity and diagnostic accuracy than leukocyte count alone (87.7% vs. 67.9%, and 83.5% vs. 67.0%, p<0.001, respectively). Negatively concordant test results had a negative predictive value of 92.1%. A simple, non-invasive OHHA assay has significantly higher specificity for acute appendicitis than the leukocyte count and concordant OHHA and leukocyte count results provides significantly higher specificity and diagnostic accuracy than leukocyte count alone.

### EXAMPLE 10: Preparation Of An Antibody To Cysteinylated Albumin

### A. Cysteinylated HSA As The Antigen

Cysteinylatedhuman serum albumin (HSA) was prepared by incubating 1.5 mm HSA (Sigma Chemical Co.) with 5 mm cystine (Sigma Chemical Co.) for 2 hours at 40°C. The resulting product was analyzed by mass spectrometry (MS) after hydrolysis overnight and being passed through a Centricon filter (cut off of 30,000 kDa). MS analysis showed that at least three species were formed - cysteinylated HSA, cysteinylated glycated HSA and cysteinylated biglycated HSA. The solution was lyophilized and yielded approximately 700 mg cysteinylated HSA. Cysteinylated HSA can be used as the antigen for preparation of antibodies specific for cysteinylated HSA.

### B. Cysteinylated HSA Peptide As The Antigen

A cysteinylated peptide comprising amino acids of the HSA sequence on either side of Cys 34 was prepared. As noted above in Example 8, Cys 34 is the only cysteine of HSA which is not disulfide bonded (*i.e.*, it has a free thiol and can be cyseinylated). The cysteinylated peptide had the sequence Ala Gln Tyr Leu Gln Gln Cys (Cys) Pro Phe Glu Asp His Val Lys [SEQ ID NO:1]. The sequence was designed to limit the number of flanking residues so that the antibodies would be directed towards epitopes bridging the cysteinylation site.

The cysteinylated peptide was coupled to KLH, and the resulting conjugate was used as an antigen for the preparation of anti-cysteinylated HSA antibodies. To prepare the antibody, two rabbits were injected with 100 µg of the cysteinylated peptide-KLH conjugate on days 1, 28, 56 and 70. The rabbits were bled prior to each immunization. The crude antisera were tested and found to show specificity for the cysteinylated peptide.

The antisera will be purified by affinity chromatography using the cysteinylated peptide. The antibodies eluted from this column will be affinity purified using the non-cysteinylated peptide. These two affinity chromatography purifications enrich for the antibodies that react specifically with the cysteinylated peptide and deplete those that react with the unmodified peptide. To assure a purified population of antibodies, the resultant preparation will be tested by ELISA against cysteinylated peptide and non-cysteinylated peptide.

The above description of the invention, including the Examples, is intended to be merely illustrative of the invention and is not intended to limit the invention. Numerous variations, modifications and changes can be made by those skilled in the art in light of the above description without departing from the spirit and scope of the invention.

## Claims

1. A method of diagnosing appendicitis in an animal comprising the following steps:
(a) obtaining a first body fluid from the animal and obtaining a second body fluid from the animal, wherein the first and second body fluids may be the same or different;
(b) determining if the quantity of orthohydroxyhippuric acid (OHHA) present in the first body fluid of the animal is significantly elevated compared to its level in the same body fluid from normal animals;
(c) determining if the quantity of a marker of general inflammation present in the second body fluid of the animal is significantly altered compared to its level in the same body fluid from normal animals; and
(d) determining if the results obtained in steps (b) and (c) indicate the presence or absence of appendicitis.

2. The method of claim 1 wherein the first body fluid is urine, and step (b) comprises:
mixing a portion of the urine from the animal being diagnosed with a color-producing reagent that produces a color when it is contacted with OHHA;
incubating the urine and the color-producing reagent for a time sufficient to allow production of the color; and
comparing the color produced to a color comparison chart or to the colors produced by standards comprising known amounts of OHHA to determine if the quantity of OHHA present in the urine is significantly elevated.

3. The method of claim 2 wherein the color-producing reagent is ferric ions bonded to silica.

4. The method of claim 1 wherein step (b), step (c) or both are performed using a binding partner assay.

5. The method of any one of claims 1 to 4 wherein the second body fluid is urine, blood, serum or plasma.

6. The method of any one of claims 1 to 4 wherein the marker of general inflammation is interleukin 8.

7. The method of any one of claims 1 to 4 wherein the marker of general inflammation is leukocyte count or neutrophil band count.

8. The method of any one of claims 1 to 4 wherein the marker of general inflammation is a post-translationally modified protein.

9. The method of claim 8 wherein the post-translationally modified protein is cysteinylated albumin.

10. The method of any one of claims 1 to 9 wherein the animal is a human.

11. A kit for diagnosing appendicitis comprising Parts (A) and (B), wherein:
Part (A) comprises at least one container holding a reagent useful for determining if the quantity of orthohydroxyhippuric acid (OHHA) present in a body fluid of an animal is significantly elevated compared to its level in the same body fluid from normal animals; and
Part (B) comprises at least one container holding a reagent useful for determining if the quantity of a marker of general inflammation present in a body fluid of the animal is significantly altered compared to its level in the same body fluid from normal animals.

12. The kit of claim 11 wherein Part (A) comprises a container holding a color-producing reagent that produces a color when contacted with OHHA.

13. The kit of claim 11 or 12 further comprising a color comparison chart and/or one or more containers holding standards comprising known amounts of OHHA.

14. The kit of claim 11 wherein Part (B) comprises a container holding a binding partner specific for a post-translationally modified protein.

15. The kit of claim 14 wherein the post-translationally modified protein is cysteinylated albumin.

16. The kit of claim 11 wherein Part (B) comprises a container holding a binding partner specific for interleukin 8.

17. A kit for diagnosing appendicitis comprising:
at least one container holding a reagent useful for determining if the quantity of marker of general inflammation present in a body fluid of an animal is significantly altered compared to its level in the same body fluid from normal animals; and
instructions directing how the reagent is to be used to diagnose appendicitis.

18. The kit of claim 17 wherein the marker of general inflammation is a post-translationally modified protein and/or a cytokine

19. The kit of claim 18 wherein the reagent is a binding partner specific for cysteinylated albumin and/or interleukin 8.


SEQUENCE LISTING

<110> DMI BioSciences, Inc.
Bar-Or, David
Bar-Or, Raphael
Winkler, James V.
Yukl, Richard L.

<120> Diagnosis and Monitoring of Inflammation, Ischemia and Appendicitis

<130> P111052EP

<140> EP03808141.0
<141> 2003-10-02

<150> US60/417,741
<151> 2002-10-09

<160> 1

<170> PatentIn version 3.1

<210> 1
<211> 14
<212> PRT
<213> Artificial sequence

<220>
<223> artificial antigen

<400> 1

Ala Gln Tyr Leu Gln Gln Cys Pro Phe Glu Asp His Val Lys
1               5                   10